(19) Europäisches Patentamt
European Patent Office
Office européen des brevets

(11) Publication number: 0 046 349
A1

# (12) EUROPEAN PATENT APPLICATION

(21) Application number: 81303534.2

(22) Date of filing: 03.08.81

(51) Int. Cl.³: C 07 D 498/04, A 61 K 31/42
// (C07D498/04, 263/00, 205/00),(A61K31/42, 31/43),(A61K31/42, 31/545)

(30) Priority: 16.08.80 GB 8026731
10.11.80 GB 8036059

(43) Date of publication of application: 24.02.82
Bulletin 82/8

(84) Designated Contracting States: BE CH DE FR GB IT LI NL

(71) Applicant: BEECHAM GROUP LIMITED, Beecham House Great West Road, Brentford, Middlesex (GB)

(72) Inventor: Gilpin, Martin Leonard, 42 Reeve Road, Reigate Surrey (GB)

(74) Representative: Hesketh, Alan, Dr. et al, European Patent Attorney Beecham Pharmaceuticals Great Burgh Yew Tree Bottom Road, Epsom, Surrey, KT18 5XQ (GB)

## (54) Clavulanic acid derivatives, their preparation and use.

(57) The present invention provides amidines, guanidines, pseudoureas, pseudothioureas, imidates and thioimidates derived from 9-aminodeoxyclavulanic acid which has the formula:

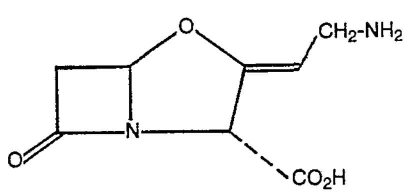

These derivatives are useful β-lactamase inhibitors and antibacterial agents. Their preparation and use are described.

EP 0 046 349 A1

Clavulanic Acid Derivatives, Their Preparation And Use

This invention relates to β-lactam antibacterial agents, to processes for their preparation and to compositions containing them.

United States Patent 4,078,067 and Belgian Patent No. 855375 relate *inter alia* to the clavulanic acid amine of the formula (I):

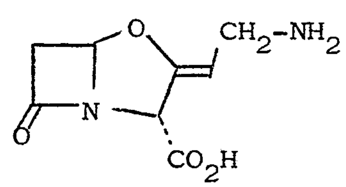

(I)

We have now found that this compound may be converted to a novel class of amidine derivatives of clavulanic acid that have good antibacterial and β-lactamase inhibitory qualities.

Accordingly the present invention provides the compounds of the formula (II):

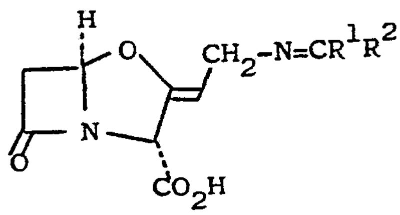

(II)

and pharmaceutically acceptable salts and esters thereof, and acid addition salts of such esters; wherein

a) $R^1$ is a group $NR^3R^4$ and $R^2$ is a group $NR^5R^6$, $SR^5$, $OR^5$ or $R^5$; or

b) $R^1$ is a group $R^5$ and $R^2$ is either a group $OR^5$ or $SR^5$;

wherein $R^3$ is selected from hydrogen, $C_{1-10}$alkyl, $C_{1-10}$ alkoxy, $C_{2-10}$ alkenyl, $C_{2-10}$ alkynyl, $C_{3-10}$ cycloalkyl, $C_{4-12}$ cycloalkylalkyl, phenyl, phenylalkyl, heteroaryl, heteroaralkyl, heterocyclyl, heterocyclylalkyl or phenylalkoxy; and $R^4$, $R^5$ and $R^6$ are independently selected from the group consisting of hydrogen, $C_{1-10}$ alkyl, $C_{2-10}$ alkenyl, $C_{2-10}$ alkynyl, $C_{3-10}$ cycloalkyl, $C_{4-12}$ cycloalkylalkyl, phenyl, phenylalkyl, heteroaryl, heteroaralkyl, heterocyclyl and heterocyclylalkyl; any of the above $R^3$, $R^4$, $R^5$ and $R^6$ groups optionally being substituted, or $R^3$ and $R^4$ may be joined together to form a heterocyclyl ring, or $R^5$ and $R^6$ may be joined together to form a heterocyclyl ring; or $R^1$ and $R^2$ may be joined together to form a heteroaryl or heterocyclyl ring: with the proviso that $OR^5$ and $SR^5$ are not OH and SH respectively.

Suitably $R^3$ is selected from hydrogen, $C_{1-10}$ alkyl, $C_{2-10}$ alkenyl, $C_{2-10}$ alkynyl, $C_{3-10}$ cycloalkyl, $C_{4-12}$ cycloalkylalkyl, phenyl, phenylalkyl, heteroaralkyl, heterocyclylalkyl, or heterocyclyl.

It is to be realised that compounds of the formula (II) may exist in the zwitterionic form (III), this being dependent on the basicity of the 9-position nitrogen atom:

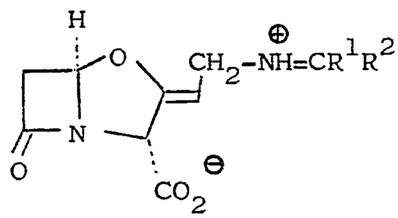

(III)

When the groups $R^3$, $R^4$, $R^5$ and $R^6$ are substituted, suitable substituents include $C_{1-6}$ alkyl, $C_{1-6}$ alkoxy, $C_{1-6}$ alkanoyl, oxo, $C_{1-6}$ alkanoyloxy, chloro, bromo, fluoro, mercapto, $C_{1-6}$ alkylthio, nitro, amino, $C_{1-6}$ alkylamino, di-$C_{1-6}$ alkylamino, hydroxy, carboxy or pharmaceutically acceptable salt or ester thereof, and cyano.

When used herein the term "heteroaryl" means a monocyclic or bicyclic ring system comprising 4 to 10 ring atoms, one, two or three of which are selected from oxygen, sulphur or nitrogen. The term "heteroaralkyl" is construed analogously with the alkyl part containing up to 6 carbon atoms. When used herein the term "heterocyclyl" means a monocyclic or bicyclic ring system comprising 4 to 10 ring atoms, one, two or three of which are selected from oxygen, sulphur or nitrogen. The term "heterocyclylalkyl is construed analogously with the alkyl part containing up to 6 carbon atoms. Similarly the groups cycloalkylalkyl, phenylalkyl and phenylalkoxy contain from one to six carbon atoms in the alkyl part.

Suitably $R^3$, $R^4$, $R^5$ and $R^6$ are independently selected from the group consisting of hydrogen, $C_{1-6}$ alkyl, $C_{3-10}$ alkenyl, phenyl, phenylalkyl and heteroaralkyl, any of the

above groups optionally being substituted by hydroxy, $C_{1-3}$ alkoxy, $C_{1-3}$ alkyl, halo, $C_{1-3}$ alkanoyloxy, or carboxy or a pharmaceutically acceptable salt or $C_{1-4}$ alkyl ester or benzyl, nitrobenzyl, $C_{1-3}$ alkylbenzyl, $C_{1-3}$ alkoxybenzyl, halobenzyl or $C_{1-3}$ alkoxycarbonylbenzyl ester thereof. In a further aspect any of $R^3$, $R^4$ and $R^5$ may be optionally substituted $C_{3-10}$ cycloalkyl, for example cyclopropyl, cyclobutyl, cyclopentyl and cyclohexyl.

Preferably $R^3$ and $R^5$ are selected from hydrogen, $C_{1-6}$ alkyl, phenyl, benzyl, $C_{1-3}$ alkoxybenzyl, $C_{1-3}$ alkylbenzyl, nitrobenzyl, chlorobenzyl, fluorobenzyl, bromobenzyl, $C_{1-3}$ alkoxycarbonylbenzyl, hydroxy $C_{1-6}$ alkyl, $C_{1-3}$ alkoxy $C_{1-6}$ alkyl, or carboxyalkyl or a pharmaceutically acceptable salt or $C_{1-4}$ alkyl ester or benzyl or nitrobenzyl ester thereof.

Preferably $R^4$ and $R^6$ are selected from hydrogen, $C_{1-6}$ alkyl or benzyl.

Suitably $R^3$ is a $C_{1-10}$ alkoxy or phenyl($C_{1-6}$)alkoxy group, for example, methoxy, ethoxy, propoxy, butoxy or benzyloxy.

One class of compounds of this invention are amidines of the formula (IV):

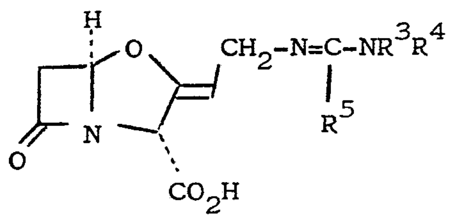

(IV)

and pharmaceutically acceptable esters thereof and pharmaceutically acceptable acid addition salts of such esters; wherein $R^3$, $R^4$ and $R^5$ are as defined hereinbefore.

Preferably in the compounds of the formula (IV), $R^3$, $R^4$ and $R^5$ are independently selected from the group consisting of hydrogen, $C_{1-6}$ alkyl such as methyl, ethyl, propyl and butyl, phenyl and benzyl. In one particularly favourable aspect $R^3$ and $R^4$ are each hydrogen.

In an alternative aspect of the compounds of the formula (IV), $R^3$ and $R^4$ may be joined to form (together with the nitrogen atom to which they are attached) a heterocyclyl ring, for example a pyrrolidino, morpholino, piperidino or azepino ring. In a further aspect either $R^3$ or $R^4$ is joined to $R^5$ to form (together with the nitrogen and carbon atoms to which they are attached) a heteroaryl or heterocyclyl ring, for example a pyrrolidinyl, morpholinyl, piperidinyl or azepinyl ring.

Thus representative examples of the groups $-N=CR^5-NR^3R^4$ for use in the compounds of this invention include:

$-N=C(CH_3)-NH_2$,
$-N=C(CH_2CH_3)-NH_2$,
$-N=C(CH(CH_3)_2)-NH_2$,
$-N=C(CH_2CH_2CH_2CH_3)-NH_2$,
$-N=C(CH_2SCH_3)-NH_2$,
$-N=C(CH_2C_4H_3S)-NH_2$,
$-N=CH-N(CH_2CH_3)_2$,
$-N=CH-N(CH_3)_2$,
$-N=C(Ph)-NHCH_3$,
-N=C(CH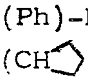)-$NH_2$,
-N=C(CH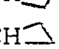)-$NH_2$,
-N=C(CH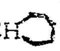)-$NH_2$,
-N=C(CH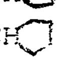)-$NH_2$,
$-N=CH-N(CH_3)_2$
$-N=C(CH_3)-NH-CH_2CH(CH_3)_2$
$-N=C(CH_3)-NH-CH_2Ph$
$-N=CH-NH_2$
$-N=CH-NH-CH_2Ph$
$-N=C(Ph)-NH_2$
$-N=C(CH_3)-NHCH_2CO_2^{\ominus}K^{\oplus}$
$-N=C(CH_3)-NH(CH_2)_5CO_2CH_2Ph$
$-N=C(CH_3)-NH(CH_2)_5CO_2^{\ominus}K^{\oplus}$
$-N=C(CH_2CH_2CH_3)-NH_2$
$-N=C(CH_2CH_2OCH_3)-NH_2$
$-N=CH-NH-CH_3$
$-N=CH-NH-CH_2CH_3$

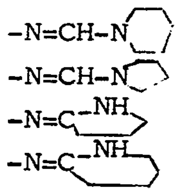

, , , ,

$-N=CH-NH-CH_2CH_2CH_3$

$-N=CH-NH-CH(CH_3)_2$

$-N=C(CH_3)-NH-CH_3$

$-N=C(CH_2CH_3)-NH-CH_3$

In another aspect of this invention there are provided guanidines of the formula (V):

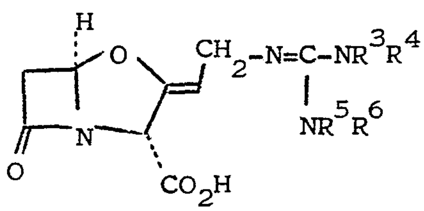

(V)

and pharmaceutically acceptable esters thereof and pharmaceutically acceptable acid addition salts of such esters, wherein $R^3$, $R^4$, $R^5$ and $R^6$ are as defined hereinbefore.

Preferably in the compounds of the formula (V), $R^3$, $R^4$, $R^5$ and $R^6$ are independently selected from the group consisting of hydrogen, $C_{1-6}$ alkyl such as methyl, ethyl, propyl and butyl, phenyl and benzyl.

Thus representative examples of the group $-N=C(-NR^3R^4)NR^5R^6$ for use in the compounds of this invention include:

$-N=C(NH_2)-NH_2$

$-N=C(NH_2)-NHCH_3$

$-N=C(NH_2)-NHCH_2CH_3$

$-N=C(NHCH_3)-NHCH_3$

$-N=C(NHCH_3)-NHCH_2CH_3$
$-N=C(NH_2)-N(CH_3)_2$
$-N=C(NHCH_3)-N(CH_3)_2$
$-N=C(NHCH_2Ph)-NH_2$
$-N=C(NHCH_2Ph)-NHCH_3$
$-N=C(NHPh)-NH_2$

In a further aspect of this invention there are provided pseudoureas and pseudothioureas of the formula(VI):

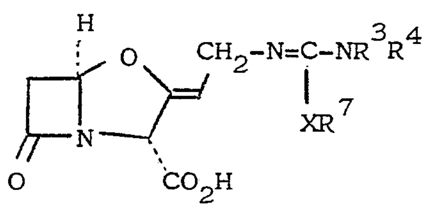

(VI)

and pharmaceutically acceptable esters thereof and pharmaceutically acceptable acid addition salts of such esters; wherein $R^3$ and $R^4$ are as defined hereinabove, $R^7$ is of the value $R^5$ except that it is not a hydrogen atom, and X is an oxygen or sulphur atom.

Preferably in the compounds of the formula (VI), $R^3$ is selected from the group consisting of $C_{1-6}$ alkyl such as methyl, ethyl, propyl and butyl, phenyl and benzyl. Preferably $R^4$ is a hydrogen atom or a methyl group.

Thus representative examples of the group $-N=C(XR^7)-NR^3R^4$ for use in the compounds of this invention include:

$-N=C(OCH_3)-NHCH_3$

$-N=C(OCH_3)-N(CH_3)_2$

$-N=C(SCH_3)-NHCH_3$

$-N=C(SCH_2CH_3)-N(CH_3)_2$

$-N=C(SCH_2CH_3)-NHPh$

$-N=C(SCH_3)-NHCH_2Ph$

In another aspect of this invention there are provided imidates and thioimidates of the formula (VII):

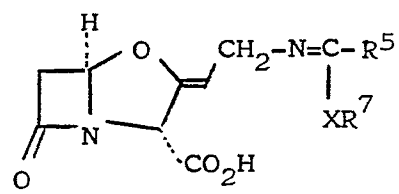

(VII)

and pharmaceutically acceptable salts and esters thereof and pharmaceutically acceptable acid addition salts of such esters; wherein $R^5$ is as defined hereinbefore, $R^7$ is of the value $R^5$ except that it is not a hydrogen atom, and X is an oxygen or sulphur atom.

Preferably in the compounds of the formula (VII), $R^5$ is a hydrogen atom, $C_{1-6}$ alkyl such as methyl, ethyl, propyl, butyl, benzyl or phenyl.

Thus representative examples of the group $-N=C(XR^7)R^5$ for use in the compounds of this invention include:

$-N{=}CH(OCH_3)$
$-N{=}C(OCH_3)CH_3$
$-N{=}C(OCH_2CH_3)CH_3$
$-N{=}C(SCH_3)Ph$

Suitable pharmaceutically acceptable salting-ions for use in salifying any carboxy group present in the groups $R^3$, $R^4$, $R^5$ and $R^6$ include alkali and alkaline earth metal salts such as the sodium, potassium, calcium and magnesium salts. Such salting-ions are also suitable for salifying the C-3 carboxyl group of the compounds of the formula (II) when they are not in zwitteronic form.

Esters of the compounds of the formula (II) also form part of thus invention, for example as the free base or as the acid addition salt since such compounds can also be used to enhance the effectiveness of penicillins or cephalosporins. Preferably such esters and addition salts are pharmaceutically acceptable.

Suitable esters of the compounds of the formula (II) include those of the formulae (VIII) and (IX):

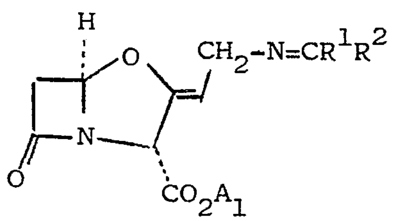

(VIII)

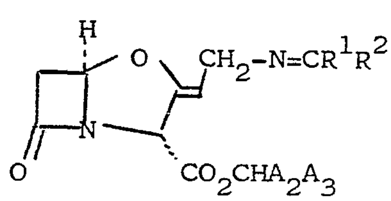

(IX)

wherein A, is $C_{1-6}$ alkyl optionally substituted by alkoxy, aryloxy, alkoxycarbonyloxy, acyloxy, aralkanoyl, alkanoyl or benzoyl of up to 8 carbon atoms, any phenyl groups in such substituents being themselves optionally substituted by a fluorine, bromine, chlorine, nitro, $C_{1-4}$ alkyl or $C_{1-4}$ alkoxy; or is a phthalidyl group optionally substituted by one or two alkyl or alkoxy groups of up to 3 carbon atoms; $A_2$ is an alkenyl or alkynyl group of up to 5 carbon atoms or is a phenyl group optionally substituted by a fluorine, chlorine, bromine, nitro or alkyl or alkoxyl of up to 4 carbon atoms; and $A_3$ is a hydrogen atom, an alkyl group of up to 4 carbon atoms or a phenyl group optionally substituted by a fluorine, chlorine, bromine, nitro or alkyl or alkoxyl of up to 4 carbon atoms.

Suitable esters of the compounds of the formula (IV) include the methyl, ethyl, n-propyl, n-butyl, allyl, 2-methylallyl, $CH_2$-CH≡CH, methoxymethyl, acetoxymethyl, propionoxymethyl, pivaloyloxymethyl, ethoxycarbonyloxymethyl, methoxycarbonyloxyethyl, ethoxycarbonyloxyethyl, phthalidyl, dimethoxyphthalidyl, benzyl, methoxybenzyl, ethoxybenzyl, nitrobenzyl and chlorobenzyl esters.

Certain favoured groups $A_1$ include methyl, methoxymethyl, acetoxymethyl, acetoxyethyl, phthalidyl, ethoxycarbonyloxymethyl, and α-ethoxycarbonyloxyethyl.

Certain favoured groups $A_2$ include the phenyl and 4-methoxyphenyl groups. A particularly favoured moiety $A_3$ is the hydrogen atom.

Esters of the compounds of the formula (II) such as those of the compounds of the formulae (IV) or (V) may be presented in the form of their acid addition salts if desired. The acid used to form the salt will most suitably be pharmaceutically acceptable, but non-pharmaceutically acceptable acid addition salts are also envisaged, for example as intermediates in the preparation of the pharmaceutically acceptable salts by ion exchange. Suitable pharmaceutically acceptable acid addition salts include those of inorganic and organic acids, such as hydrochloric, phosphoric, sulphuric, methanesulphonic, toluenesulphonic, citric, malic, acetic, lactic, tartaric, propionic and succinic acid.

Most suitably the acid addition salt is provided as a solid and preferably as a crystalline solid.

The present invention also provides a pharmaceutical composition which comprises a compound of the formula (II) or a pharmaceutically acceptable salt or ester thereof or a pharmaceutically acceptable acid addition salt of such an ester and a pharmaceutically acceptable carrier.

Most suitably the composition of this invention will contain the free acid of a compound of the formula (II).

The compositions of the invention include those in a form adapted for oral, topical or parenteral use and may be used for the treatment of infection in animals for example mammals including humans.

Suitable forms of the compositions of this invention include tablets, capsules, creams, syrups, suspensions, solutions, reconstitutable powders and sterile forms suitable for injection or infusion. Such compositions may contain conventional pharmaceutically acceptable materials such as diluents, binders, colours, flavours, preservatives, disintegrant and the like in accordance with conventional pharmaceutical practice in the manner well understood by those skilled in the art of formulating antibiotics.

Injectable or infusable compositions of a compound of the invention are particularly suitable as high blood levels of the compound can occur after administration by injection or infusion. Thus, one preferred composition aspect of this invention comprises a compound of the invention in sterile form and most suitably in sterile crystalline form.

The injectable solution of the compound of this invention may be made up in a sterile pyrogen-free liquid such as water, aqueous ethanol or the like.

An alternative approach to administering the compounds of this invention is to utilise an injectable suspension. Such suspensions may be made up in sterile water; sterile saline or the like, and may also contain suspending agents such as polyvinylpyrrolidone, lecithin or the like. Alternatively such compositions may be prepared in an acceptable oil suspending agent such as arachis oil or its equivalent. For use in such suspensions the compounds of this invention should be in the form of fine particles.

Unit dose compositions comprising a compound of this invention adapted for oral administration form a further suitable composition aspect of this invention.

Unit dose compositions comprising a compound of this invention adapted for topical administration are also presented by this invention. In this instance 'topical administration' also includes local administration to internal surfaces of mammary glands of cattle, for example during the treatment of mastitis by intra-mammary administration.

The compound of the invention may be present in the composition as sole therapeutic agent or it may be present together with other therapeutic agents such as a penicillin or cephalosporin. Considerable advantages accrue from the inclusion of a penicillin or cephalosporin which shows instability to β-lactamases since the resulting composition shows enhanced effectiveness (synergy). Suitable penicillins, cephalosporins or other β-lactam antibiotics for inclusion in such synergistic compositions include not only those

known to be highly susceptible to β-lactamases but also those which have a degree of intrinsic resistance to β-lactamases.

Suitable penicillins for inclusion in the compositions of this invention include benzylpenicillin, phenoxymethylpenicillin, carbenicillin, azidocillin, propicillin, ampicillin, amoxycillin, epicillin, ticarcillin, cyclacillin, pirbenicillin, azlocillin, mezlocillin, celbenicillin, pipericillin, and other known penicillins including pro-drugs therefor such as their *in vivo* hydrolysable esters such as the acetoxymethyl, pivaloyloxymethyl, α-ethoxycarbonyloxyethyl or phthalidyl esters of ampicillin, benzylpenicillin or amoxycillin, and aldehyde or ketone adducts of penicillins containing a 6-α-aminoacetamide side chain (such as hetacillin, metampicillin and analogous derivatives of amoxycillin) or α-esters of carbenicillin or ticarcillin such as their phenyl or indanyl α-esters.

A further group suitable penicillins of interest for inclusion in the compositions of this invention include benzylpenicillin, phenoxymethylpenicillin, carbenicillin, azidocillin, propicillin, amoxycillin, epicillin, ticarcillin, cyclacillin, pirbenicillin, azlocillin, mezlocillin, celbenicillin, pipericillin, and other known penicillins including pro-drugs therefor such as their in vivo hydrolysable esters such as the acetoxymethyl, pivaloyloxymethyl, α-ethoxycarbonyloxyethyl or phthalidyl esters of benzylpenicillin or amoxycillin, and α-esters of carbenicillin or ticarcillin such as their phenyl or indanyl α-esters.

Suitable cephalosporins for inclusion in the compositions of this invention include cefatrizine, cephaloridine, cephalothin, cefazolin, cephalexin, cephacetrile, cephamandole nafate, cephapirin, cephradine, 4-hydroxycephalexin, cefaparole, cephaloglycin, cefoperazone and other known cephalosporins or pro-drugs thereof.

Such compounds are frequently used in the form of a salt or hydrate of the like.

Naturally if the penicillin or caphalosporin present in the composition is not suitable for oral administration then the composition will be adapted for parenteral administration.

Highly favoured penicillins for use in the compositions of this invention include ampicillin,amoxycillin, carbenicillin and ticarcillin. Such penicillins may be used as a pharmaceutically acceptable salt such as the sodium salt. Alternatively the ampicillin or amoxycillin may be used in the form of find particles of the zwitterionic form (generally as ampicillin trihydrate or amoxycillin trihydrate) for use in an injectable suspension, for example in the manner hereinbefore described for a compound of this invention.

The preferred penicillin for use in the synergistic composition is amoxycillin, for example as its sodium salt or trihydrate.

Particularly suitable cephalosporins for use in the compositions of this invention include cephaloridine and cefazolin which may be in the form of a pharmaceutically acceptable salt for example the sodium salt.

When present together with a cephalosporin or penicillin, the ratio of a compound of the invention to the penicillin or cephalosporin agent may vary over a wide range of ratios, such as from 10:1 to 1:10 for example about 3:1, 2:1, 1:1, 1:2, 1:3, 1:4, 1:5 or 1:6, (wt/wt, based on pure free antibiotic equivalent). Orally administrable compositions containing a compound of the invention will normally contain relatively more synergist than corresponding injectable compositions.

The total quantity of a compound of the invention in any unit dosage form will normally be between 25 and 1000 mg and will usually be between 50 and 500 mg, for example about 62.5, 100, 125, 150, 200 or 250 mg.

Compositions of this invention may be used for the treatment of infections of animals for example mammals including humans, such infections being _inter alia_ of the respiratory tract, the urinary tract and soft tissues in humans and mastitis in cattle.

Normally between 50 and 3000 mg of the compounds of the invention will be administered each day of treatemnt but more usually between 100 and 1000 mg of the compounds of the invention will be administered per day, for example at 1-6 doses, more usually as 2, 3 or 4 doses. However, for the treatment of more severe systemic infections or infections of particularly intransigent organisms higher doses may be used in accordance with clinical practice.

The penicillin or cephalosporin in the synergistic composition of this invention will normally be present at approximately the amount at which it is conventionally used which will usually be expected to be from about 62.5 to 3000 mg per dose, more usually about 125, 250, 500 or 1000 mg per dose.

One particularly favoured composition of this invention will contain from 150 to 1000 mg of amoxycillin as the trihydrate or sodium salt and from 25 to 500 mg of a compound of this invention.

A further particularly favoured composition of this invention will contain from 150 to 1000 mg of ampicillin or a pro-drug therefor and from 25 to 500 mg of a compound of this invention.

Most suitably this form of composition will contain ampicillin trihydrate, ampicillin anhydrate, sodium ampicillin, hetacillin, pivampicillin hydrochloride, bacampicillin hydrochloride, or talampicillin hydrochloride. Most suitably this form of the composition will contain a compound of this invention when in crystalline form.

Most suitably the preceding composition will contain from 200 to 700 mg of the penicillin component. Most suitably the preceding composition will comprise from 50 to 250 mg of a compound of this invention preferably in crystalline form.

Such compositions may be adapted for oral or parenteral use except when containing an <u>in vivo</u> hydrolysable ester of ampicillin or amoxycillin in which case the compositions will not be adapted for parenteral administration.

Another particularly favoured composition of this invention will contain from 200 to 2000 mg of carbenicillin or ticarcillin or a pro-drug therefor and from 50 to 500 mg of a compound of the invention.

Suitably this form of composition will contain di-sodium carbenicillin. Suitably this form of the composition will contain di-sodium ticarcillin.

More suitably this form of the composition will contain from 75 to 250 mg of a compound of this invention preferably in crystalline form. Such compositions containing di-salts of carbenicillin and ticarcillin will be adapted for parenteral administration.

The present invention also provides a method of treating bacterial infection in animals such as humans or domestic mammals which comprises the administration of a composition of this invention.

Commonly the infection treated will be due to a strain of Staphylococcus aureus, Klebsiella aerogenes, Escherichia coli, Proteus sp., Bacteroides fragilis or the like. The organisms believed to be most readily treated by an antibacterially effective amount of a compound of this invention is Staphylococcus aureus. The other organisms named are more readily treated by using a synergistically effective amount of the compound of the invention and a penicillin or cephalosporin. The administration of the two components may take place separately but in general we prefer to use a composition containing both the synergist and the penicillin or cephalosporin.

The indications for treatment include respiratory tract and urinary tract infections in humans and mastitis in cattle.

In a further aspect the present invention provides a process for the preparation of the compounds of the formulae (IV) and (V) and esters thereof and pharmaceutically acceptable acid addition salts of such esters, which process comprises the reaction of a compound of the formula (X) or a derivative thereof that allows alkylation to occur:

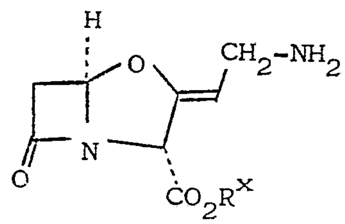

(X)

wherein $R^x$ is a hydrogen atom or a blocking group; with either the compound of the formula (XI) or (XII):

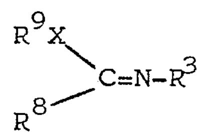

(XI)

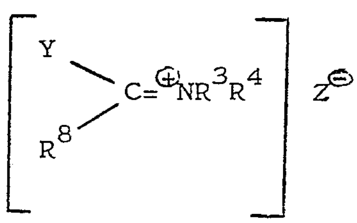

(XII)

wherein $R^8$ is a group $R^5$ or $NR^5R^6$; $R^9$ is $C_{1-6}$ alkyl, X is an oxygen or sulphur atom, Y is halo or $C_{1-6}$ alkoxy, and Z is a salting-ion, and $R^3$ and $R^4$ are as defined in relation to formula (IV), and thereafter if necessary removing the blocking group. The compound of the formula (XI) is preferably used in the form of its acid addition salt, for example as the hydrochl

An example of a derivative that allows alkylation to occur is a silyl derivative, for example, the trimethyl.

Suitably $R^9$ is a methyl or ethyl group. Suitably Y is halo such as a chloro atom. Suitably also Y is $C_{1-6}$ alkoxy such as methoxy or ethoxy. Suitably Z is halo such as chloro, or is a tetrafluoroborate ion. Suitably X is an oxygen atom.

Reaction of the compound of the formula (X) with either the compound of the formula (XI) or (XII) is suitably performed in water, ether, dioxan, tetrahydrofuran,dimethylformamide, dichloromethane or mixtures of such solvents. Suitably the reaction is performed between $0^oC$ and ambient temperature. In aqueous media we have found it convenient to carry out the reaction at a basic pH, for example 7.5 to 10, preferably 8 to 9.

However, when Y is $C_{1-6}$ alkoxy in the compound of the formula (XII) the reaction is suitably performed in an inert organic solvent, such as a chlorinated solvent, for example dichloromethane or chloroform; using approximately one equivalent of an organic base of low nucleophilicity, for example diazabicyclononene.

In another aspect the present invention provides a process for the preparation of compounds of the formulae (IV) and (V) and esters thereof and pharmaceutically acceptable acid addition salts of such esters, which process comprises the reaction of a compound of the formula (XIII):

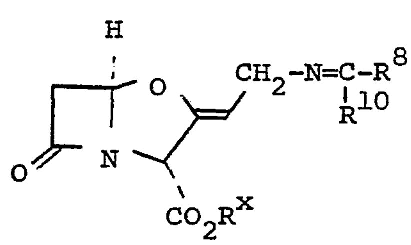

(XIII)

wherein $R^x$ and $R^8$ are as hereinbefore defined and $R^{10}$ is chloro or a group $XR^9$ as hereinbefore defined; with a compound of the formula (XIV):

$$HNR^3R^4 \qquad (XIV)$$

wherein $R^3$ and $R^4$ are as hereinbefore defined, and thereafter if necessary removing the blocking group.

The reaction conditions utilised are similar to those for the reaction of the compound of the formula (X) and the compound of the formula (XI).

In another aspect of this invention there is provided a process for the preparation of the compound of the formula (VI) and esters thereof and pharmaceutically acceptable acid addition salts of such esters, which process comprises the reaction of a compound of the formula (XV):

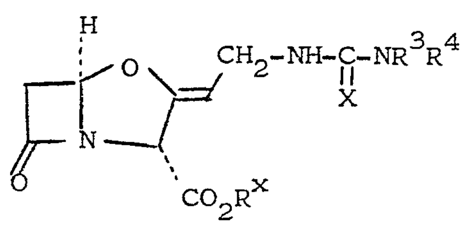

(XV)

wherein $R^x$, $R^3$, $R^4$ and X are as hereinbefore defined, and a reactive alkylating agent, and thereafter if necessary removing the blocking-group.

Suitable alkylating agents include methyl iodide, ethyl iodide, benzyl bromide, dimethylsulphate, allylbromide and methyl chloromethyl ether. Suitable solvents include alcohols, dioxan and acetonitrile at temperature of ambient to 60°C.

In a further aspect of the this invention there is provided a process for the preparation of a compound of the formula (VII) and pharmaceutically acceptable salts and esters thereof and pharmaceutically acceptable acid addition salts of such esters, which process comprises the reaction of a compound of the formula (XVI):

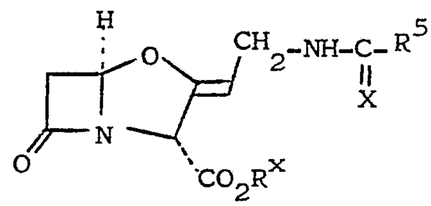

(XVI)

wherein $R^x$, X, and $R^5$ are as hereinbefore defined with a reactive alkylating agent, and thereafter if necessary removing the blocking group.

Suitable alkylating agents include triethyloxonium tetrafluoroborate, trimethyloxonium tetrafluoroborate, dimethyl sulphate and methyl fluorosulphonate. Suitably the reaction is performed at a depressed temperature for example -80°C to 0°C, in inert organic solvents such as dichloromethane, chloroform, tetrahydrofuran and dioxan.

Suitable carboxyl-blocking derivatives for the group $-CO_2R^x$ in formula (X), (XV) and (XVI) include salts and ester derivatives of the carboxylic acid. The derivative is preferably one which may readily be cleaved at a later stage of the reaction. Suitable salts include metal salts, such as those with sodium, potassium and lithium, and tertiary amine salts, such as those with tri-lower-alkylamines, N-ethylpiperidine, 2,6-lutidine, pyridine, N-methylpyrrolidine, dimethylpiperazine. A preferred salt is with triethylamine.

Suitable ester-forming carboxyl-blocking groups are those which may be removed under conventional conditions. Such groups for $R^x$ include benzyl, p-methoxybenzyl, 2,4,6-trimethylbenzyl, 3,5-di-t-butyl-4-hydroxybenzyl, benzoylmethyl, p-nitrobenzyl, 4-pyridylmethyl, 2,2,2-trichloroethyl, 2,2,2-tribromoethyl, t-butyl, t-amyl, diphenylmethyl, triphenylmethyl, adamantyl, 2-benzyloxyphenyl, 4-methylthiophenyl, tetrahydrofur-2-yl, tetrahydropyran-2-yl, pentachlorophenyl, p-toluenesulphonylethyl, methoxymethyl, a silyl, stannyl or phosphorus-containing group, an oxime radical of formula $-N=CHR^o$ where $R^o$ is aryl or heterocyclic, or an <u>in-vivo</u> hydrolysable ester radical such as defined above. It will be appreciated that it is possible that any carboxyl-blocking group present may be converted to another carboxyl-blocking group during the course of the reaction.

The carboxyl group may be regenerated from any of the above esters by usual methods appropriate to the particular $R^x$ group, for example, acid - and base - catalysed hydrolysis, or by enzymically - catalysed hydrolysis, or by hydrogenation.

The compounds of the formulae (XV) and (XVI) may be prepared by the methods of Belgian Patent Nos. 860042 and 866496.

A pharmaceutically acceptable ester of a compound of the formula (II) may be prepared by reaction of the compound of the formula (II) with an esterifying agent.

The zwitterionic compound of the formula (II) may be dissolved or suspended in a solvent such as dimethylformamide, hexamethylphosphoramide, dichloromethane, ethyl acetate or other non-esterifiable solvents and therein esterified. Suitable temperatures for such a reaction range from about $0^{o}$ to about $25^{o}C$. Suitable esterifying reagents include reactive halides and their equivalents and alkyl oxonium salts.

When a reagent such as a reactive iodide,chloride, bromide, tosylate, mesylate or the equivalent is used, the resulting salt is generally suitable for use in a composition of this invention. Alternatively, the salt may be converted to a free base or alternative salt. When an alkyl oxonium salt is used, it is preferred to convert the resulting tetrafluoroborate to the free base or alternative salt. The various afore-mentioned salts may be converted to the free base by neutralisation, for example by contacting a solution of the salt in water with an organic phase, neutralising the salt by adding a base and extracting the liberated amine into the organic phase. This amine may thereafter be re-salted by reacting with an appropriate acid, for example in a dry organic solvent. It is generally preferred to use not more than one equivalent of acid for this process. Alternatively, the originally formed salt may be converted into the alternative salt using an ion exchange material for example, by passing an aqueous solution of one salt through a bed of an

anion exchange resin in the form of the form of the desired salt such as the chloride form.

The salts may normally be obtained in solid form by dissolving in a farly polar organic solvent (such as ethanol or tetrahydrofuran) and then precipitating using a less-polar solvent such as diethyl ether or cyclohexane.

The salts of the esters of the compounds of the formula (II) may normally be obtained in crystalline form by conventional methods such as trituration under (or crystallisation or recrystallisation form) a suitable organic solvent such as ether, acetone, acetonitrile or tetrahydrofuran.

Esters of the compound of the formula (II) may also be prepared by reaction of an acid addition salt of the compound of the formula (II) with an alcohol in the presence of a condensation promoting agent.

Suitable condensation promoting agents for use in this process include carbodiimides such as dicyclohexyl-carboxiimide and the chemical equivalents thereof.

The acid addition salt may be formed in situ or may be preformed. The acid employed will normally be a strong acid such as a methane sulphonic acid, p-toluene sulphonic acid or trifluoroacetic acid.

The reaction is normally carried out in an inert organic solvent. When the ester being formed is that of a liquid alcohol it is convenient to use that alcohol as the solvent or as part of the solvent system. The esterification is generally performed at a non-extreme temperature such as $0^{o}$ to $35^{o}C$, for example from about $10^{o}$ to $25^{o}C$. Conveniently the reaction may be performed at ambient temperature.

The compounds of the formula (XIII) wherein $R^{10}$ is chloro may be prepared via the reaction of a compound of the formula (XVI) with a halogenated agent such as phophorus pentachloride, thionylchloride, phosgene, phosphorus trichloride or phosphorus oxychloride, in the presence of a base, such as triethylamine, pyridine or N-methylmorpholine.

The compounds of this formula (XIII) wherein $R^{10}$ is a group $XR^{9}$ may be prepared by the reaction of a compound of the formula (XIII) wherein $R^{10}$ is chloro, with a $C_{1-6}$ alkanol or $C_{1-6}$ alkylmercaptan in the presence of a base such as triethylamine, pyridine or N-methylmorpholine.

The compounds of the formula (XIII) are novel and as such form part of this invention.

The following Examples illustrate the invention. In the Examples 9-ADCA represents 9-aminodeoxyclavulanic acid, DMF represents N,N-dimethylformamide, DBN represents diazabicyclononene, THF represents tetrahydrofuran, ETOAC represents ethyl acetate, MDC represents dichloromethane, and dil HCl represents dilute hydrochloric acid.

Example 1

9-N-(Dimethylaminomethylene)amino-9-deoxyclavulanic acid

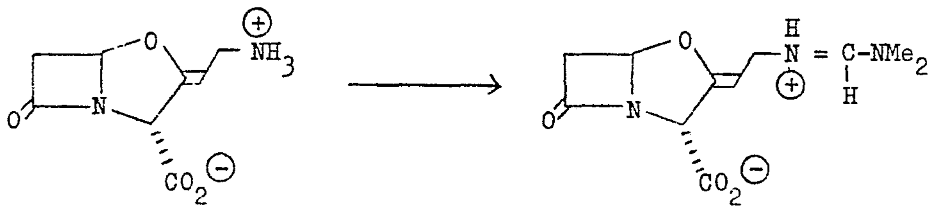

9-ADCA (198 mg, 1.0 mmol) in dry DMF (10 ml) was cooled to 0° and the stirred suspension treated with DBN (375 mg, 3.0 mmol). After 2 mins. the solution was treated with trimethylchlorosilane (0.13 ml, 1.0 mmol) and stirring continued for a further 5 mins. Methyl 4-bromocrotonate (0.75 ml) was now added to the solution and stirring at 0° continued for 30 mins. After a further 2½ hrs at room temperature the reaction mixture was evaporated to dryness.

The residue was chromatographed on silica gel. Elution with n-butanol/ethanol/water (4:1:1) gave a little recovered 9-ADCA and further elution with (2:1:1) afforded the desired amidine as an oil (73 mg, 29%).

$\upsilon_{max}$ (liq. film) 1790, 1700, 1620 $cm^{-1}$.

$\delta$($D_2O$) 2.92 (3H, s, $NC\underline{H}_3$), 3.13 (3H, s, $NC\underline{H}_3$), β-lactam C$\underline{H}$H obscured, 3.52 (1H, dd, $\underline{J}$ 17 and 3 Hz, β-lactam CH$\underline{H}$), 4.00 (2H, d, $\underline{J}$ 7 Hz, C(9)$\underline{H}$), 4.75 (1H, t, $\underline{J}$ 7 Hz, vinyl $\underline{H}$), 4.90 (1H, s, C(3)$\underline{H}$), 5.69 (1H, d, $\underline{J}$ 3 Hz, β-lactam C$\underline{H}$), 7.72 (1H, s, C$\underline{H}$) ppm.

Example 2

9-N-(1'-aminoethylidene)amino-9-deoxyclavulanic acid

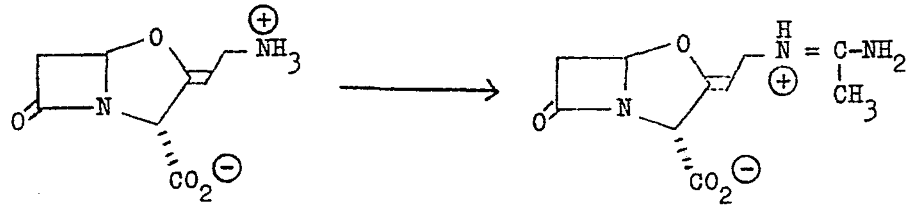

9-ADCA (198 mg, 1.0 mmol) in distilled water (15 ml) was cooled to 0° and the stirred solution adjusted to pH 8.5 by the addition of 1N LiOH solution dispensed from an automatic burette. Ethyl acetimidate hydrochloride (800 mg, 6.5 mmol) was then added to the solution in small portions over 5 mins, the pH of the solution being maintained at 8.5 throughout. After the addition stirring at 0° was continued for a further 45 mins, and then the pH of the solution adjusted to 7.0 by the addition of dil. HCl.

Evaporation of the solvent under reduced pressure gave a crude product which was chromatographed on silica gel. Elution with n-butanol/ethanol/water (4:1:1) gave the desired product as a white solid (143 mg, 60%).

$\nu_{max}$ (KBr) 1790, 1690, 1620 (broad) $cm^{-1}$.

$\delta(D_2O)$ 2.14 (3H, s, $CH_3$), 3.03 (1H, d, J 17 Hz, β-lactam CHH), 3.55 (1H, dd, J 17 and 3 Hz, β-lactam CHH), 3.89 (2H, d, J 7 Hz, C(9)H), 4.77 (1H, t, J 7 Hz, vinyl H), 4.90 (1H, s, C(3)H), 5.68 (1H, d, J 3 Hz, β-lactam CH) ppm.

Example 3

9-N-[1'-(N-isobutyl)aminoethylidene]amino-9-deoxyclavulanic acid

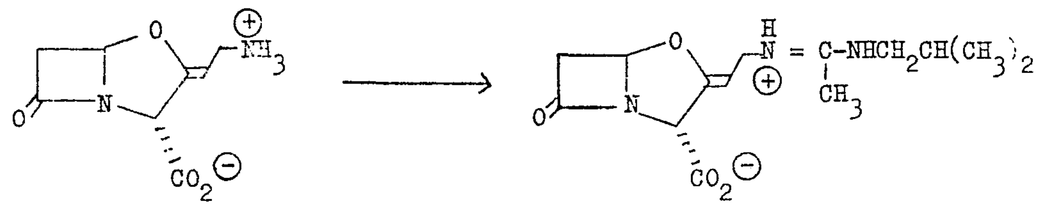

9-ADCA (198 mg, 1.0 mmol) in distilled water (15 ml) was cooled to 0° and the stirred solution adjusted to pH 8.5 by the addition of 1N LiOH solution dispensed from an automatic burette. Ethyl N-isobutylacetimidate (0.50 g, 3.5 mmol) in THF (2 ml) was added to the above solution over 2 mins, and the pH of the solution allowed to rise to 9.0. Stirring at 0° was continued for 1 hr and the pH then adjusted to 7.0 by the addition of dil. HCl.

Evaporation of the solvent afforded an oil which was chromatographed on silica gel. Elution with n-butanol/ethanol/water (4:1:1) gave the desired product as a white solid (56 mg, 19%).

$\upsilon_{max}$ (KBr) 1790, 1690, 1660, 1610 $cm^{-1}$.

$\delta(D_2O)$ 0.86 (6H, d, J 7 Hz, $CHMe_2$), 1.82 (1H, m, $CHMe_2$), 2.16 and 2.2. (3H, s, $C-CH_3$), 2.98 and 3.16 (2H, d, J 7 Hz, $NCH_2CH$), 3.04 (1H, d, J 17 Hz, β-lactam CHH), 3.58 (1H, dd, J 17 and 3 Hz, β-lactam CHH), 3.91 and 3.98 (2H, d, J 7 Hz, C(9)H), 4.76 (1H, t, J 7 Hz, vinyl H), 4.89 and 4.91 (1H, s, C(3)H), 5.73 (1H, d, J 3 Hz, β-lactam CH) ppm. Isomer ratio ~3:2.

Example 4

9-N-[1-(N-benzyl)aminoethylidene]amino-9-deoxyclavulanic acid

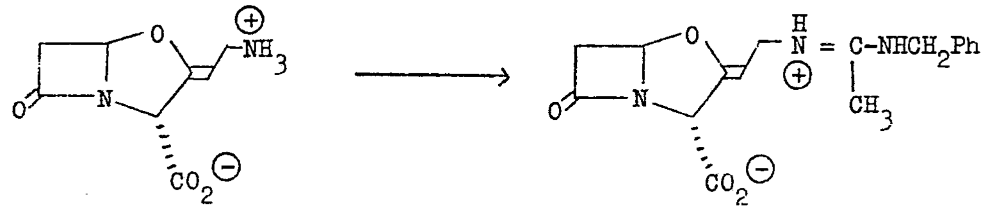

9-ADCA (198 mg, 1.0 mmol) in distilled water (15 ml) was cooled to 0° and the sitrred solution adjusted to pH 8.5 by the addition of 1N LiOH solution. Ethyl N-benzylacetimidate (3.79 g, 21.4 mmol) in THF (5 ml) was added to the above solution over 2 mins and the pH of the solution allowed to rise to 9.0. It was necessary to add THF to the reaction mixture to ensure complete solution of the acetimidate. The solution was stirred at 0° for 1 hr and then the pH adjusted to 7.0 by addition of dil. HCl.

The THF was evaporated under reduced pressure and the residue partitioned between water and EtOAc. The aqueous layer was evaporated to afford an oil which was chromatographed on silica gel. Elution with n-butanol/ethanol/water (4:1:1) gave the desired product as a white solid (188 mg, 57%).

$\upsilon_{max}$ (KBr) 1790, 1690, 1650, 1610, 740 $cm^{-1}$.

$\delta$($D_2O$) 2.13 and 2.23 (3H, s, C-C$\underline{H}_3$), 2.82 and 2.90 (1H, d, $\underline{J}$ 17 Hz, β-lactam C$\underline{H}$H), 3.39 and 3.47 (1H, dd, $\underline{J}$ 17 and 3 Hz, β-lactam CH$\underline{H}$), 3.86 and 3.96 (2H, d, $\underline{J}$ 7 Hz, C(9)$\underline{H}$), 4.38 and 4.52 (2H, s, NC$\underline{H}_2$Ph), 4.71 (1H, t, $\underline{J}$ 7 Hz, vinyl H), 4.85 and 4.88 (1H, s, C(3)$\underline{H}$), 5.43 and 5.62 (1H, d, $\underline{J}$ 3 Hz, β-lactam C$\underline{H}$), 7.33 (5H, s, Aryl $\underline{H}$) ppm. Isomer ratio ~3:2.

Example 5

9-N-(aminomethylene)amino-9-deoxyclavulanic acid

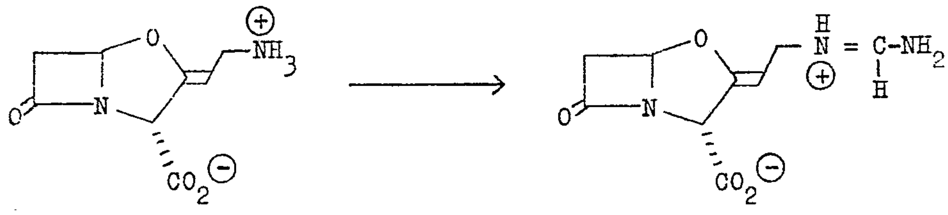

9-ADCA (198 mg, 1.0 mmol) in distilled water (15 ml) was stirred and cooled to 0°. The pH of the reaction mixture was maintained at 8.5 (by the addition of 1M LiOH solution dispensed from an automatic burette) during the addition of ethyl formimidate hydrochloride (1.10 g, 10.0 mmol) in small portions. After the addition the solution was stirred at 0° for 1 hr and then the pH adjusted to 7.0 by addition of dil. HCl.

Evaporation of the water under reduced pressure afforded an oil which was chromatographed on silica gel. Elution with n-butanol/ethanol/water (4:1:1 → 2:1:1) afforded the desired product as a pale yellow solid (101 mg, 45%).

$\upsilon_{max}$ (KBr) 1785, 1700, 1610 $cm^{-1}$.

$\delta$($D_2O$) 3.05 (1H, d, J 17 Hz, β-lactam CHH), 3.52 (1H, dd, J 17 and 3 Hz, β-lactam CHH), 3.92 and 3.99 (2H, d, J 7 Hz, C(9)H), 4.80 (1H, t, J 7 Hz vinyl H), 4.93 (1H, s, C(3)H), 5.71 (1H, d, J 3 Hz, β-lactam CH), 7.71 (1H, s, CH = N) ppm.

Example 6

9-N-(N-benzylaminomethylene)amino-9-deoxyclavulanic acid

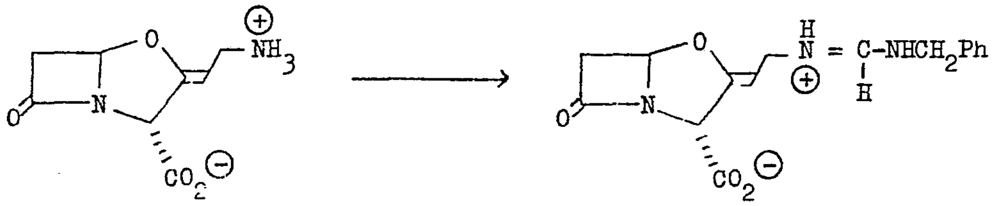

9-ADCA (198 mg, 1.0 mmol) in distilled water (15 ml) was stirred at 0°. The pH of the reaction mixture was maintained at 8.5 (by the addition of 1M LiOH solution dispensed from an automatic burette) during the addition of a solution of ethyl N-benzylformimidate hydrofluoroborate (3.43 g, 13.6 mmol) in THF (10 ml). It was found necessary to add further THF to the reaction mixture from time to time in order to keep the formimidate in solution. After addition was complete the homogenous solution was stirred for a further 1 hr at 0° and pH 8.5.

The pH was then adjusted to 7.0 by addition of dil. HCl and the THF evaporated under reduced pressure. The aqueous suspension was washed well with EtOAc and evaporated to afford an oil which was chromatographed on silica gel. Elution with n-butanol/ethanol/water (4:1:1) gave the desired product as a white solid (200 mg, 64%).

$\upsilon_{max}$ (KBr) 1790, 1700, 1620, 740 $cm^{-1}$.

$\delta(D_2O)$ 2.80 and 2.95 (1H, d, *J* 17 Hz, β-lactam C<u>H</u>H), 3.39 and 3.49 (1H, dd, *J* 17 and 3 Hz, β-lactam CH<u>H</u>), 3.90 and 3.98 (2H, d, *J* 7 Hz, C(9)<u>H</u>), 4.41 and 4.52 (2H, s, $NCH_2Ph$), 4.78 (1H, t, *J* 7 Hz, vinyl <u>H</u>), 4.85 (1H, s, C(3)<u>H</u>), 5.49 and 5.61 (1H, d, *J* 3 Hz, β-lactam C<u>H</u>), 7.33 (5H, aryl <u>H</u>), 7.86 (1H, s, N=C<u>H</u>) ppm. Isomer ratio 1:1.

Example 7

9-N-(1-aminobenzylidene)amino-9-deoxyclavulanic acid

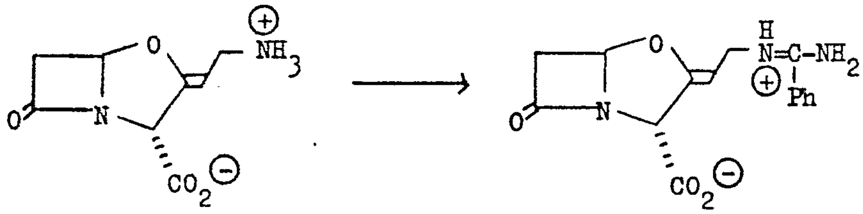

A solution of 9-ADCA (100 mg, 0.50 mmol) in distilled water (15 ml), containing DMF (10 ml), was cooled to 0° and the stirred solution adjusted to pH 9.0 by the addition of 1M LiOH solution. Ethyl benzimidate hydrochloride (1.38 g, 7.5 mmol) in a little water was added portionwise to the above solution over 10 mins, the pH of the reaction mixture being maintained at 9.0 by addition of 1N LiOH solution dispensed from an automatic burette. The pH was finally adjusted to 9.5 and stirring at 0° maintained for 1½ hours. Neutralisation to pH 7.0 by addition of dil. HCl solution followed by evaporation of the solvents under reduced pressure afforded the crude product which was chromatographed on silica gel. Elution with n-butanol/ethanol/water (4:1:1) gave the desired product as a white solid (135 mg, 89%).

$\upsilon_{max}$ (nujol) 1790, 1680, 1610 $cm^{-1}$.

δ($D_2O$) 3.03 (1H, d, J 17Hz, β-lactam CHH), 3.52 (1H, dd, J 17 and 3 Hz, β-lactam CHH), 4.06 (2H, d, J 7 Hz, C(9)H), 4.88 (1H, t, J 7 Hz, vinyl H), 4.95 (1H, s, C(3) H), 5.72 (1H, d, J 3 Hz, β-lactam CH), 7.60 (5H, s, ArH) ppm.

Example 8

9-N-[1-(N-5'-benzyloxycarbonylpentyl)aminoethylidene]amino-9-deoxyclavulanic acid

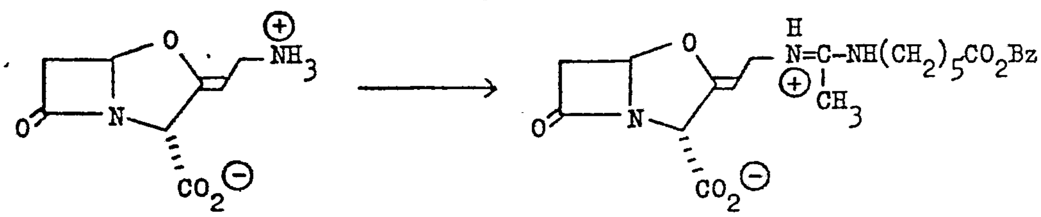

A solution of 9-ADCA (200 mg, 1.0 mmol) in distilled water (40 ml) was stirred at 0°. Ethyl N-(5-benzyloxycarbonylpentyl)acetimidate (3.72 g, 13 mmol) in THF (60 ml) was added to the above solution in small portions whilst the pH was maintained at 8.5 throughout. The pH was now adjusted to 9.5 by addition of 1M LiOH solution and stirring at 0° continued for a further 2 hrs. The reaction mixture was then neutralised to pH 7.0 by addition of dil. HCl and the solvents evaporated.

Chromatography on silica gel eluting with n-butanol/ethanol/water (4:1:1) gave the desired product as a white solid (233 mg, 53%).

$\upsilon_{max}$ (nujol) 1790, 1730, 1650, 1600 $cm^{-1}$.

$\delta$($D_2O$) 1.5 (6H, overlapping signals, $C\underline{H}_2$), 2.08 and 2.13 (3H, s, $C\underline{H}_3$), 2.28 (2H, t, J 7 Hz, $C\underline{H}_2CO_2$), 2.95 (1H, d, J 17 Hz, β-lactam C<u>H</u>H), 3.05 (2H, t, J 7 Hz, $NC\underline{H}_2$), 3.48 (1H, overlapping dd, β-lactam CH<u>H</u>), 3.80 and 3.95 (2H, d, J 7 Hz, C(9)<u>H</u>), 4.75 (1H, t, J 7 Hz, vinyl <u>H</u>), 4.87 (1H, s, C(3)<u>H</u>), 5.00 (2H, s, $C\underline{H}_2Ph$), 5.68 (1H, d, J 3 Hz, β-lactam C<u>H</u>), 7.25 (5H, s, aryl <u>H</u>) ppm. Isomer ratio 1:1.

Example 9

Lithium 9-N-[1-(N-5'-Carboxylatopentyl)aminoethylidene]amino-9-deoxyclavulanic acid

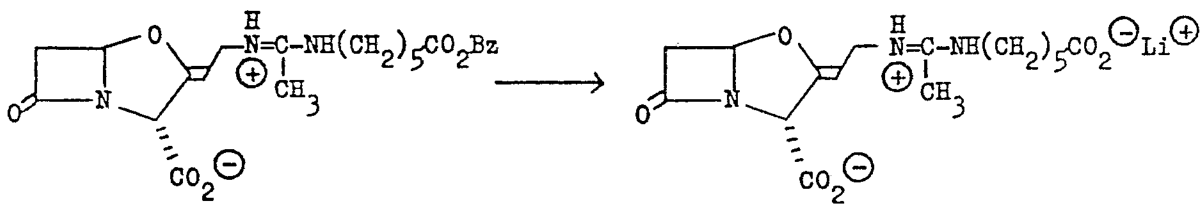

The benzyl ester (100 mg) in aq. THF (20 ml) was hydrogenated over 10% Pd/C (50 mg) for 30 mins at ambient temperature and pressure. The catalyst was filtered off and the resulting solution neutralised to pH 7.0 by addition of dil. LiOH solution. The solvents were removed under reduced pressure and the residue chromatographed on silica gel. Elution with n-butanol/ethanol/water (1:1:1) gave the desired product as a white solid (17 mg, 21%).

$\upsilon_{max}$ (KBr) 1780, 1660, 1600, 1560 $cm^{-1}$.

$\delta$($D_2O$) 1.5 (6H, overlapping signals, $C\underline{H}_2$), 2.14 (2H, t, $\underline{J}$ 7 Hz, $C\underline{H}_2CO_2^{\ominus}$), 2.20 (3H, s, $C\underline{H}_3$), 2.90 (1H, d, $\underline{J}$ 17 Hz, β-lactam C$\underline{H}$H), 3.18-3.50 (3H, overlapping signals, $NC\underline{H}_2$ and β-lactam CH$\underline{H}$), 4.00 (2H, d, $\underline{J}$ 7 Hz, C(9)$\underline{H}$), vinyl $\underline{H}$ obscurred by HOD, 4.95 (1H, s, C(3)$\underline{H}$), 5.70 (1H, d, $\underline{J}$ 3 Hz, β-lactam C$\underline{H}$) ppm.

Example 10

Potassium 9-N-[1'-(N-Carboxylatomethyl)aminoethylidene]amino-9-deoxyclavulanic acid

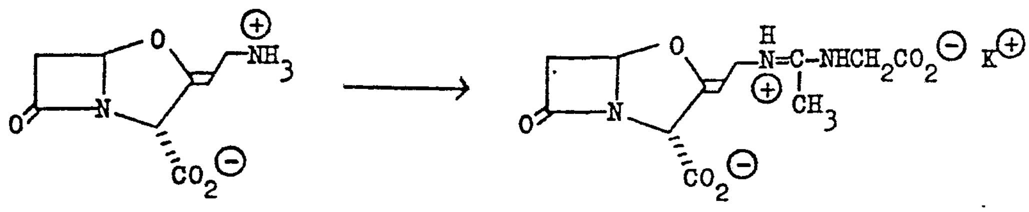

A solution of 9-ADCA (198 mg, 1.0 mmol) in distilled water (10 ml) was stirred at 0° and the pH adjusted to 8.5 by addition of dil. KOH solution. Potassium ethyl N-carboxylatomethylacetimidate (1.41 g, 7.7 mmol) was now added in small portions and the pH constantly adjusted to 8.5. Stirring at 0° was then continued for a further 1 hr. After this time the pH was adjusted to 7.0 by the addition of dil. HCl and the solution evaporated to dryness.

The residue was chromatographed on silica gel eluting with n-butanol/ethanol/water (2:1:1). After 3 such chromatographic purifications the product was obtained as a white solid (42 mg, 13%).

$\upsilon_{max}$ (KBr) 1790, 1660, 1600 $cm^{-1}$.

$\delta$($D_2O$) 2.15 and 2.22 (3H, s, $C\underline{H}_3$), 3.06 (1H, d, $\underline{J}$ 17 Hz, β-lactam $C\underline{H}H$), 3.55 (1H, dd, $\underline{J}$ 17 and 3 Hz, β-lactam $CH\underline{H}$), 3.92 (2H, d, $\underline{J}$ 7 Hz, C(9)$\underline{H}$), 3.91 (2H, s, $NC\underline{H}_2CO_2$), 4.83 (1H, t, $\underline{J}$ 7 Hz, vinyl $\underline{H}$), 4.96 (1H, s, C(3)$\underline{H}$), 5.73 (1H, d, $\underline{J}$ 3 Hz, β-lactam $C\underline{H}$) ppm. Isomer ratio ca 1:1.

Example 11

9-N-(N-methylaminomethylene)amino-9-deoxyclavulanic acid

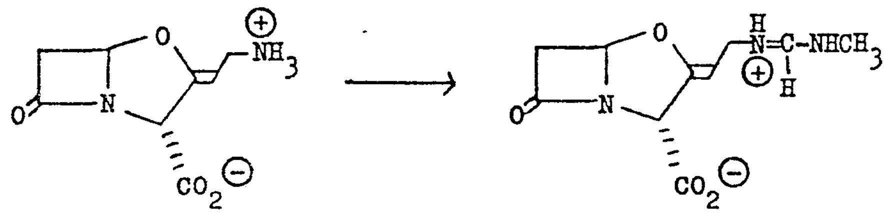

A solution of 9-ADCA (200 mg, 1.0 mmol) in distilled water (15 ml) was cooled to 0° and stirred. Ethyl N-methylformimidate hydrogen tetrafluoroborate (3.5 g, 19.0 mmol) in water (10 ml) was added in small portions to the above solution whilst the pH was maintained at 8.5 by addition of 1M NaOH solution dispensed from an automatic burette. The solution was stirred at this pH and 0° for a further 1 hr. and then neutralised to pH 7.0 by addition of dil. HCl. The solvents were removed under reduced pressure.

Chromatography on silica gel, eluting with n-butanol/ethanol/water (4:1:1) afforded the desired product as a white solid (32 mg, 13%).

$\upsilon_{max}$ (KBr) 1790, 1700, 1620 $cm^{-1}$.

$\delta$($D_2O$) 2.80 and 3.02 (3H, s, $NC\underline{H}_3$), 3.05 (1H, d, $\underline{J}$ 17 Hz, β-lactam C$\underline{H}$H), 3.51 (1H, dd, $\underline{J}$ 17 and 3 Hz, β-lactam CH$\underline{H}$), 3.86 and 4.02 (2H, d, $\underline{J}$ 7 Hz, C(9)$\underline{H}$), 4.80 (1H, t, $\underline{J}$ 7 Hz, vinyl $\underline{H}$), 4.90 (1H, s, C(3)$\underline{H}$), 5.70 (1H, d, $\underline{J}$ 3 Hz, β-lactam C$\underline{H}$), 7.62 and 7.73 (1H, s, C$\underline{H}$) ppm. Isomer ratio 2:1.

Example 12

9-N-(1-aminobutylidene)amino-9-deoxyclavulanic acid

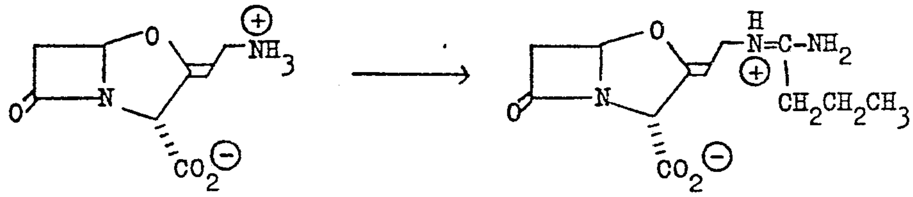

A solution of 9-ADCA (400 mg, 2.0 mmol) in water (30 ml) was stirred at 0° and treated portionwise with a solution of ethyl butyrimidate hydrogen tetrafluoroborate (3.76 g, 18.6 mmol) in water (20 ml). During the addition the pH of the solution was maintained at 8-9 by addition of 1M NaOH solution dispensed from an automatic burette. The solution was then stirred at pH 9.0 and 0° for 2 hours. The reaction mixture was neutralised to pH 7.0 by addition of dil. HCl and the solvent evaporated.

The residue was chromatographed on silica gel eluting with n-butanol/ethanol/water (2:1:1) to afford the desired product as a white solid (312 mg, 59%).

$\upsilon_{max}$ (nujol) 1790, 1690, 1600 $cm^{-1}$.

$\delta(D_2O)$ 0.90 (3H, t, J 7 Hz, $CH_2C\underline{H}_3$), 1.65 (2H, m, $CH_2C\underline{H}_2CH_3$), 2.42 (2H, t, J 7 Hz, $C\underline{H}_2CH_2$), 3.08 (1H, d, J 17 Hz, β-lactam C$\underline{H}$H), 3.56 (1H, dd, J 17 and 3 Hz, β-lactam CH$\underline{H}$), 3.90 (2H, d, J 7 Hz, C(9)$\underline{H}$), 4.82 (1H, t, J 7 Hz, vinyl $\underline{H}$), 4.93 (1H, s, C(3)$\underline{H}$), 5.72 (1H, d, J 3 Hz, β-lactam C$\underline{H}$) ppm.

Example 13

9-N-(1'-amino-3'-methoxypropylidene)amino-9-deoxyclavulanic acid

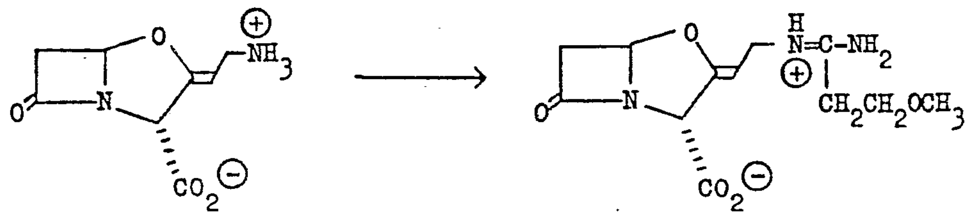

A solution of 9-ADCA (200 mg, 1.0 mmol) in water (15 ml) was stirred at 0° and the pH adjusted to 8.5 by addition of 1M NaOH solution. A solution of ethyl 3-methoxypropionimidate hydrochloride (0.90 g, 5.4 mmol) in water (15 ml) was added portionwise whilst the pH was maintained at 8-9 by addition of 1M NaOH solution dispensed from an automatic burette. The reaction mixture was stirred at 0° and pH 9.5 for a further 1 hr and then neutralised to pH 7.0 by addition of dil. HCl. The solvent was evaporated under reduced pressure.

Chromatography on silica gel, eluting with n-butanol/ethanol/water (4:1:1), afforded the desired product as a white solid (235 mg, 83%).

$\upsilon_{max}$ (KBr) 1790, 1690, 1620 $cm^{-1}$.

$\delta$($D_2O$) 2.74 (2H, t, $\underline{J}$ 7 Hz, N=C-C$\underline{H}_2$$CH_2$), 3.12 (1H, d, $\underline{J}$ 17 Hz, β-lactam C$\underline{H}$H), 3.35 (3H, s, OC$\underline{H}_3$), 3.60 (1H, dd, $\underline{J}$ 17 and 3 Hz, β-lactam CH$\underline{H}$), 3.72 (2H, t, $\underline{J}$ 7 Hz, $CH_2$C$\underline{H}_2$O), 3.95 (2H, d, $\underline{J}$ 7 Hz, C(9)$\underline{H}$), 4.86 (1H, t, $\underline{J}$ 7 Hz, vinyl $\underline{H}$), 4.98 (1H, s, C(3)$\underline{H}$), 5.76 (1H, d, $\underline{J}$ 3 Hz, β-lactam C$\underline{H}$) ppm.

Example 14

9-N-(1'-aminopropylidene)amino-9-deoxyclavulanic acid

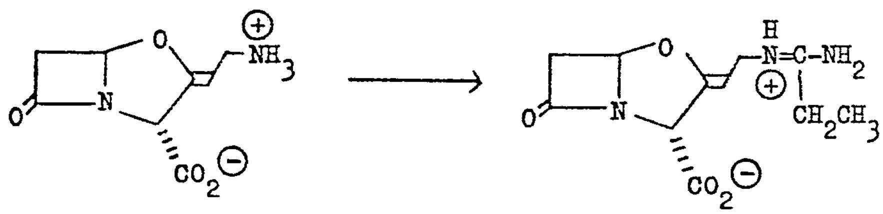

A solution of 9-ADCA (200 mg, 1.0 mmol) in water (20 ml) was stirred at 0° and treated portionwise with a solution of ethyl propionimidate hydrogen tetrafluoroborate (1.13 g, 6.0 mmol) in water (15 ml). Throughout the addition the pH was maintained at 8-8.5 by the addition of 1M NaOH solution. The reaction mixture was then stirred at 0° and pH 8.5 for a further 1½ hr, and subsequently neutralised to pH 7.0 by addition of dil. HCl. The solvent was evaporated under reduced pressure.

Chromatography on silica gel, eluting with n-butanol/ethanol/water (2:1:1), gave the desired product as a white solid (150 mg, 59%).

$\upsilon_{max}$ (KBr) 1790, 1690, 1620 $cm^{-1}$.

$\delta$($D_2O$) 1.13 (3H, t, J 7 Hz, $CH_2CH_3$), 2.39 (2H, q, J 7 Hz, $CH_2CH_3$), 3.00 (1H, d, J 17 Hz, β-lactam CHH), 3.48 (1H, dd, J 17 and 3 Hz, β-lactam CHH), 3.85 (2H, d, J 7 Hz, C(9)H), 4.75 (1H, t, J 7 Hz, vinyl H), 4.86 (1H, s, C(3)H), 5.67 (1H, d, J 3 Hz, β-lactam CH)ppm.

Example 15

9-N-(Dimethylaminomethylene)amino-9-deoxyclavulanic acid

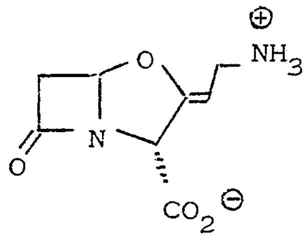

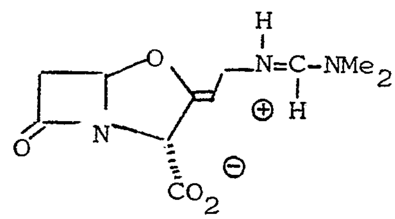

A suspension of 9-aminodeoxyclavulanic acid (200 mg, 1.0 mmol) in DMF (10 ml) was cooled to $0^{o}$ and stirred during the addition of DBN (126 mg, 1.0 mmol) followed by ethyl N, N-dimethylformimidate tetrafluoroborate (186 mg, 1 mmol). Stirring at $0^{o}$ was maintained for a further 1 hr. and the solution then allowed to warm to room temperature and stirred for a further 2 hrs. The solvent was then evaporated under reduced pressure.

Chromotography on silica gel eluting with n-butanol/ethanol/water (2:1:1) gave the desired product as a white solid (132 mg, 52%).

Example 16

9-N-(1'-amino-2'-methyl propylidene) amino -9- deoxyclavulanic acid.

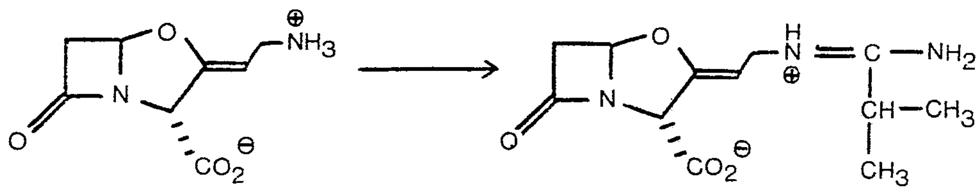

A solution of 9-ADCA (200 mgs) in water (20 mls) was stirred at 0°C and treated with ethyl isobutyrimidate hydrogen tetrafluoroborate (1g) added portionwise. During the addition the pH of the solution was maintained at 9 with 1M NaOH solution dispensed from an automatic burette. Stirring at pH 9 and 0°C was continued for 1 hour before a further addition of the imidate (1g) maintaining a pH of 9. Stirring at pH 9 and 0°C was continued for 1½ hours. The reaction mixture was neutralised with dil HCl solution before evaporating the solvent in vacuo.

The residue was chromatographed on silica gel eluting with n-butanol/ethanol/ water (2:1:1) to afford the desired product (61 mgs, 23%).

$\nu_{max}$ (KBr) 1790, 1700, 1640, 1605 $cm^{-1}$.

$\delta$ ($D_2O$) 1.16 (6H, d, J 7 Hz, $CH_3$), 2.67 (1H, m, J 7 Hz, CH $Me_2$), 3.01 (1H, d, J 17Hz, β-lactam CHH), 3.50 (1H, dd, J 17 and 3Hz, β-lactam CHH), 3.89 (2H, d, J 7Hz, C (9) H), 4.79 (1H, t, J 7Hz, vinyl H), 4.90 (1H, s, C (3) H), 5.70 (1H, d, J 3Hz, β-lactam CH) ppm.

Example 17

9-N-(1'-amino-3'-methyl butylidene) amino-9-deoxyclavulanic acid.

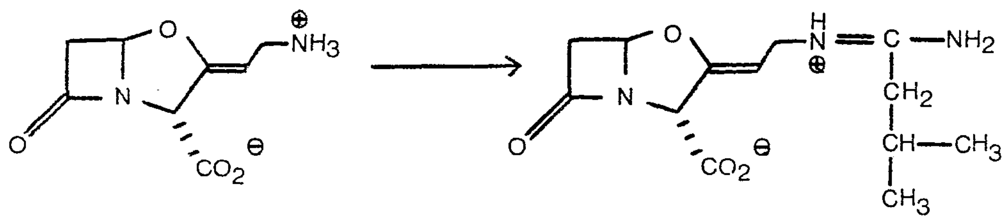

A solution of 9-ADCA (200 mgs) in water (25 mls) was stirred at 0°C and treated with ethyl isovalerimidate hydrogen tetrafluoroborate (1.75g) added portionwise. During the addition the pH of the solution was maintained at 8.5-9 with 1M LiOH solution dispensed from an automatic burette. The solution was then stirred at pH 9 and 0°C for 2 hours. The reaction mixture was neutralised to pH 7 with dil HCl solution before evaporating the solvent in vacuo.

The residue was chromatographed on silica gel eluting with n-butanol/ethanol/water (4:1:1) to afford the desired product (52 mgs, 18%).

$\nu_{max}$ (KBr) 1780, 1700, 1680, 1610 cm $^{-1}$.

δ ($D_2O$) 0.90 (6H, d, J 7Hz, $CH_3$), 1.94 (1H, m, CH $Me_2$), 2.26 (2H, d, J 7Hz, $N{=}CCH_2$), 3.02 (1H, d, J 17Hz, β-lactam CHH), 3.53 (1H, dd, J 17 and 3Hz, β-lactam CHH), 3.87 (2H, d, J 7Hz, C (9) H), 4.82 (1H, t, J 7Hz, vinyl H), 4.90 (1H, s, C (3) H), 5.70 (1H, d, J 3Hz, β-lactam CH) ppm.

Example 18

9-N-(1-amino pentylidene) amino-9-deoxyclavulanic acid.

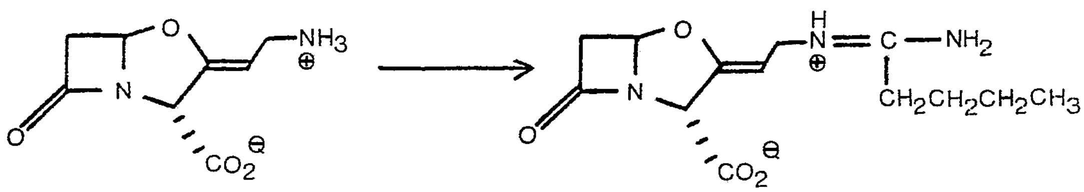

A solution of 9-ADCA (200 mgs) in water (25 mls) was stirred at 0°C and treated with ethyl pentanimidate hydrochloride (1.4g) added portionwise. During the addition the pH of the solution was maintained at pH 9 with 1M LiOH solution dispensed from an automatic burette. The solution was then stirred at pH 9 and 0°C for 1½ hours. The reaction mixture was neutralised to pH 7 with dil HCl solution before evaporating the solvent in vacuo.

The residue was chromatographed on silica gel eluting with ethanol, then rechromatographed using n-butanol/ethanol/water (4:1:1) to afford the desired product (162 mgs, 57%).

$\nu_{max}$ (KBr) 1780, 1700, 1670, 1620 cm $^{-1}$.

δ ($D_2O$) 0.90 (3H, t, _J_ 7Hz, $C\underline{H}_3$), 1.30 - 1.70 (4H, overlapping m, $C\underline{H}_2$), 2.46 (2H, t, _J_ 7Hz, $N{=}CC\underline{H}_2$), 3.10 (1H, d, _J_ 17Hz, β-lactam $C\underline{H}H$), 3.58 (1H, dd, _J_ 17 and 3Hz, β-lactam $CH\underline{H}$), 3.93 (2H, d, _J_ 7Hz, C (9) $\underline{H}$), 4.87 (1H, t, _J_ 7Hz, vinyl $\underline{H}$), 4.97 (1H, s, C (3) $\underline{H}$), 5.77 (1H, d, _J_ 3Hz, β-lactam $C\underline{H}$) ppm.

Example 19

9-N-(amino-cyclopropylmethylene) amino-9-deoxyclavulanic acid.

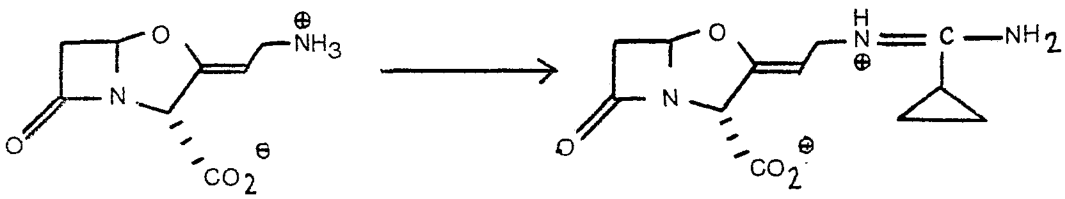

A solution of 9-ADCA (200 mgs) in water (25 mls) was stirred at 0°C and treated with ethyl cyclopropylcarboximidate hydrogen tetrafluoroborate (1.6g) added portionwise. During the addition the pH of the solution was maintained at 8.5-9 with 1M LiOH solution dispensed from an automatic burette. The solution was then stirred at pH 9 and 0° for 2 hours before evaporating the solvent in vacuo.

The residue was chromatographed on silica gel eluting with n-butanol/ethanol/water (4:1:1) to afford the desired product (64 mgs, 24%).

$\nu_{max}$ (KBr) 1780, 1690, 1610 cm $^{-1}$.

δ ($D_2O$) 1.0 - 1.2 (4H, overlapping m, C$\underline{H}_2$), 1.82 (1H, m, N=CC$\underline{H}$), 3.09 (1H, d, $\underline{J}$ 17Hz, β-lactam C$\underline{H}$H), 3.58 (1H, dd, $\underline{J}$ 17 and 3Hz, β-lactam CH$\underline{H}$), 3.93 (2H, d, $\underline{J}$ 7Hz, C (9) $\underline{H}$), 4.82 (1H, t, $\underline{J}$ 7Hz, vinyl $\underline{H}$), 4.96 (1H, s, C (3) $\underline{H}$), 5.75 (1H, d, $\underline{J}$ 3Hz, β-lactam C$\underline{H}$) ppm.

Example 20

9-N-(amino-cyclobutylmethylene) amino-9-deoxyclavulanic acid.

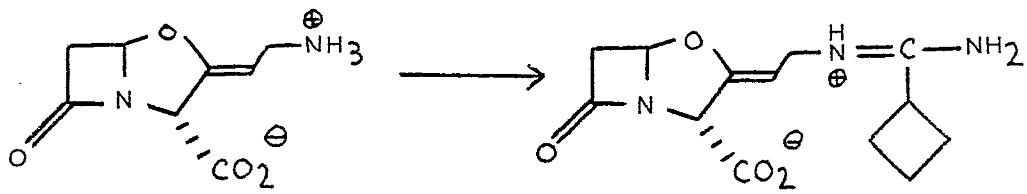

A solution of 9-ADCA (200 mgs) in water (25 mls) was stirred at 0°C and treated with ethyl cyclobutyl carboximidate hydrogen tetrafluoroborate (1.75g) added portionwise. During the addition the pH of the solution was maintained at 8.5-9 with 1M LiOH solution dispensed from an automatic burette. The solution was then stirred at pH 9 and 0° for 2 hours. The reaction mixture was neutralised to pH 6.5 with dil HCl solution before evaporating the solvent in vacuo.

The residue was chromatographed on silica gel eluting with n-butanol/ethanol/water (2:1:1) to afford the required product (105 mgs, 38%).

$\nu_{max}$ (KBr) 1780, 1700, 1680, 1610 cm $^{-1}$.

δ ($D_2O$) 1.8 - 2.3 (6H, overlapping m, C$\underline{H}_2$), 3.03 (1H, d, $\underline{J}$ 17Hz, β-lactam C$\underline{H}$H), 3.25 (1H, m, N=CC$\underline{H}$), 3.53 (1H, dd, $\underline{J}$ 17 and 3Hz, β-lactam CH$\underline{H}$), 3.89 (2H, d, $\underline{J}$ 7Hz, C (9) $\underline{H}$), 4.78 (1H, t, $\underline{J}$ 7Hz, vinyl $\underline{H}$ ), 4.92 (1H, s, C (3) $\underline{H}$), 5.72 (1H, d, $\underline{J}$ 3Hz, β-lactam C$\underline{H}$) ppm.

Example 21

9-N-(diethyl amino methylene) amino-9-deoxyclavulanic acid.

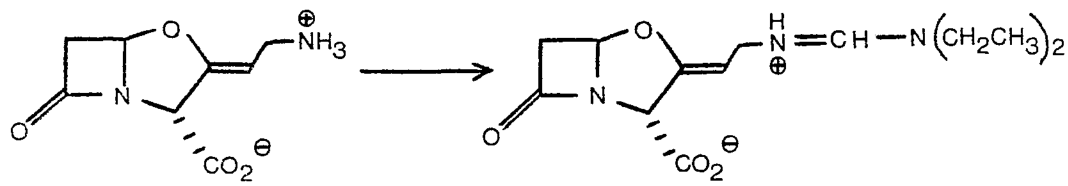

A suspension of 9-ADCA (200 mgs) in M.D.C. (20 mls) was stirred at 0°C during the addition of DBN (140 mgs) followed by ethyl N, N-diethylformimidate tetrafluoroborate (217mgs). The solution was allowed to warm to room temperature and stirring continued for 2½ hours. The solvent was then evaporated in vacuo.

The residue was chromatographed on silica gel eluting with n-butanol/ethanol/water (1:1:1) to afford the desired product (68mgs, 24%).

δ ($D_2O$) 1.11 (3H, t, J 7 Hz, $CH_2CH_3$), 1.19 (3H, t, J 7 Hz, $CH_2CH_3$), 3.00 (1H, d, J 17 Hz, β-lactam CHH), 3.30 (2H, q, J 7Hz, $CH_2CH_3$), 3.42 (2H, q, J 7 Hz, $CH_2CH_3$), 3.51 (1H, dd, J 17 and 3Hz, β-lactam CHH), 3.97 (2H, d, J 7Hz, C (9) H), 4.78 (1H, t, J 7Hz, vinyl H), 4.88 (1H, s, C (3) H), 5.68 (1H, d, J 3 Hz, β-lactam CH), 7.70 (1H, s, N=CH) ppm.

Example 22

9-N-[1-(N,N-dimethyl) amino ethylidene] amino-9-deoxyclavulanic acid.

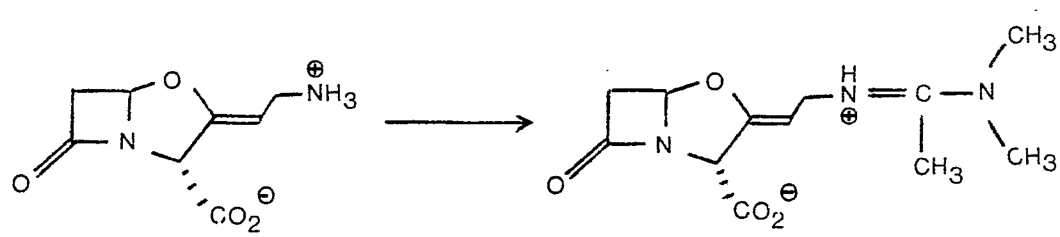

A suspension of 9-ADCA (200 mgs) in MDC (25 mls) was stirred at 0°C during the addition of DBN (137 mgs) followed by ethyl N, N-dimethylacetimidate tetrafluoroborate (203 mgs). The solution was allowed to warm to room temperature and stirring continued for 2 hours. The solvent was then evaporated in vacuo.

The residue was chromatographed on silica gel eluting with n-butanol/ethanol/ $H_2O$ (2:1:1) to afford the required product (63 mgs, 24%).

$\nu_{max}$ (KBr) 1790, 1690, 1640, 1610 $cm^{-1}$.

δ ($D_2O$) 2.25 (3H, s, $CC\underline{H}_3$), 3.07 (1H, d, $\underline{J}$ 17Hz, β-lactam $C\underline{H}H$), 3.08 (3H, s, $NC\underline{H}_3$), 3.18 (3H, s, $NC\underline{H}_3$), 3.57 (1H, dd, $\underline{J}$ 17 and 3Hz, β-lactam $CH\underline{H}$), 4.07 (2H, d, $\underline{J}$ 7Hz, C (9) $\underline{H}$), 4.78 (1H, t, $\underline{J}$ 7Hz, vinyl $\underline{H}$), 4.95 (1H, s, C (3) $\underline{H}$), 5.75 (1H, d, $\underline{J}$ 3Hz, β-lactam $C\underline{H}$) ppm.

Example 23

9-N-[1'-(N-methyl) amino benzylidene] amino-9-deoxyclavulanic acid.

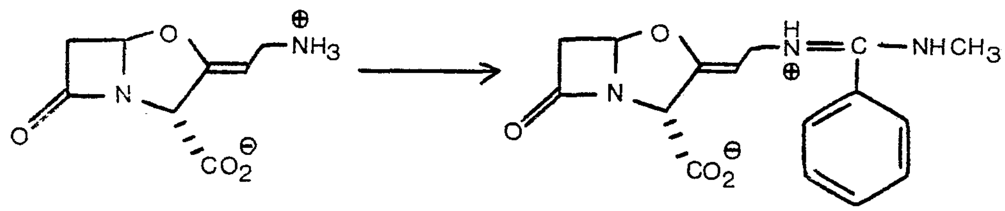

A solution of 9-ADCA (200 mgs) in water (20 mls) was stirred at 0°C and treated with a solution of ethyl N-methylbenzimidate hydrogen tetrafluoroborate (1.5g) in D.M.F. (15 mls) added portionwise. During the addition the pH of the solution was maintained at 9 with 1M NaOH solution dispensed from an automatic burette. Stirring at pH 9 and 0°C was continued for 1½ hours. The reaction mixture was neutralised with dil HCl solution before evaporating the solvent in vacuo.

The residue was chromatographed on silica gel eluting with n-butanol/ethanol/water (2:1:1) to afford the desired product (48mgs 15%).

$\nu_{max}$ (KBr) 1790, 1690, 1650, 1610 cm $^{-1}$.

δ ($D_2O$) 2.80 (1H, d, *J* 17Hz, β-lactam CHH), 3.01 (3H, s, $CH_3$), 3.42 (1H, dd, *J* 17 and 3Hz, β-lactam CHH), 3.91 and 4.05 (2H, d, *J* 7Hz, C (9) H), 4.75 (1H, t, *J* 7Hz, vinyl H), 4.90 and 4.95 (1H, s, C (3) H), 5.45 and 5.75 (1H, d, *J* 3Hz, β-lactam CH), 7.5 (5H, m, aryl H) ppm. Isomer ratio 2:1.

Example 24

9-N-[1-(N-methyl) amino ethylidene] amino-9-deoxyclavulanic acid.

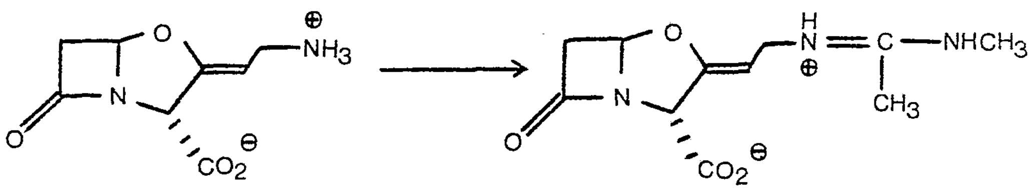

A solution of 9-ADCA (200mgs) in water (20 mls) was stirred at 0°C and treated with ethyl N-methylacetimidate hydrogen tetrafluoroborate (1.13g) in water (20mls) added portionwise. During the addition the pH of the solution was maintained at 8.5-9 with IM NaOH solution dispensed from an automatic burette. The solution was then stirred at pH 9 and 0°C for 1 hour. The reaction mixture was neutralised to pH 7 with dil HCl solution before evaporating the solvent in vacuo.

The residue was chromatographed on silica gel eluting with n-butanol/ethanol/water (2:1:1) to afford the desired product (125mgs; 49%).

$\nu_{max}$ (KBr) 1785, 1700, 1660, 1610 cm $^{-1}$.

δ ($D_2O$) 2.12 and 2.16 (3H, s, C-C$\underline{H}_3$), 2.77 and 2.92 (3H, s, N-C$\underline{H}_3$), 3.00 (1H, d, $\underline{J}$ 17Hz, β-lactam C$\underline{H}$H), 3.50 (1H, dd, $\underline{J}$ 17 and 3Hz, β-lactam CH$\underline{H}$), 3.80 and 3.98 (2H, d, $\underline{J}$ 7Hz, C(9) $\underline{H}$), 4.73 (1H, t, $\underline{J}$ 7Hz, vinyl $\underline{H}$), 4.88 (1H, s, C (3) $\underline{H}$), 5.67 (1H, d, $\underline{J}$ 3Hz, β-lactam C$\underline{H}$) ppm. Isomer ratio 3:2.

Example 25

9-N-[1-(N-ethyl) amino ethylidene] amino-9-deoxyclavulanic acid.

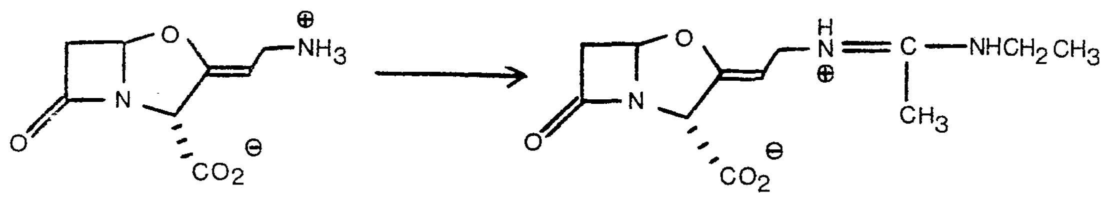

A solution of 9-ADCA (200mgs ) in water (20 mls) was stirred at 0°C and treated with ethyl N-ethyl acetimidate hydrogen tetrafluoroborate (1.23g) in water (15 mls) added portionwise. During the addition the pH of the solution was maintained at 8.5-9 with 1M NaOH solution, dispensed from an automatic burette. The solution was then stirred at pH 9.5 and 0°C for 1 hour. The reaction mixture was neutralised to pH 7 with dil HCl solution before evaporating the solvent in vacuo.

The residue was chromatographed on silica gel eluting with n-butanol/ethanol/water (2:1:1) to afford the desired product (136 mgs 51%).

$\nu_{max}$ (KBr) 1790, 1700, 1660, 1610 cm $^{-1}$.

δ ($D_2O$) 1.11 (3H, t, J 7Hz, $CH_2C\underline{H}_3$), 2.14 (3H, s, $C\underline{H}_3$), 3.00 (1H, d, J 17Hz, β-lactam $C\underline{H}H$), 3.39 (2H, q, J 7Hz, $C\underline{H}_2CH_3$), 3.48 (1H, dd, J 17 and 3Hz, β-lactam $CH\underline{H}$), 3.80 and 3.95 (2H, d, J 7Hz, C (9) $\underline{H}$), 4.71 (1H, t, J 7Hz, vinyl $\underline{H}$), 4.85 (1H, s, C (3) $\underline{H}$), 5.66 (1H, d, J 3Hz, β-lactam $C\underline{H}$) ppm. Isomer ratio 3:2.

Example 26

9-N-(N,N-pentamethylene amino methylene) amino-9-deoxyclavulanic acid.

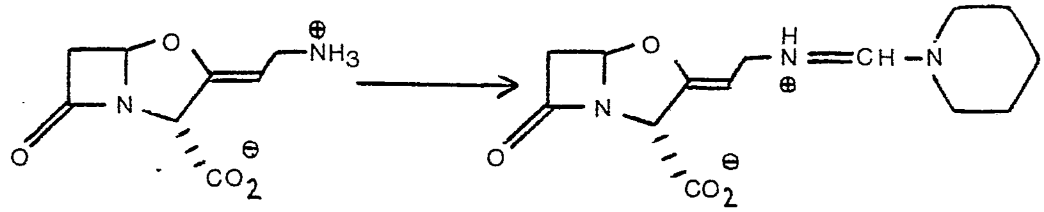

A suspension of 9-ADCA (200 mgs) in MDC (30 mls) was stirred at 0°C during the addition of DBN (140 mgs) followed by N,N-pentamethylene formimidate tetrafluoroborate (230 mgs). Stirring at 0°C was continued for 1½ hours before a further addition of DBN (70 mgs) and the formimidate (115 mgs). The solution was allowed to warm to room temperature and stirring continued for 2 hours. The solvent was then evaporated in vacuo. The residue was chromatographed on silica gel eluting with n-butanol/ethanol/water (2:1:1) to afford the desired product (45 mgs, 15%).

$\nu_{max}$ (KBr) 1790, 1700, 1690, 1610 $cm^{-1}$.

δ ($D_2O$) 1.54 (6H, overlapping m, $C\underline{H}_2$), 2.95 (1H, d, $\underline{J}$ 17 Hz, β-lactam C$\underline{H}$H), ca. 3.3 (4H, t, $\underline{J}$ 7Hz, $NC\underline{H}_2$), 3.45 (1H, dd, $\underline{J}$ 17 and 3Hz, β-lactam CH$\underline{H}$), 3.90 (2H, d, $\underline{J}$ 7Hz, C (9) $\underline{H}$), 4.73 (1H, t, $\underline{J}$ 7Hz, vinyl $\underline{H}$), 4.88 (1H, s, C (3) $\underline{H}$), 5.67 (1H, d, $\underline{J}$ 3Hz, β-lactam C$\underline{H}$), 7.65 (1H, s, N= C$\underline{H}$) ppm.

Example 27

9-N-(N,N-hexamethylene amino methylene) amino-9-deoxyclavulanic acid.

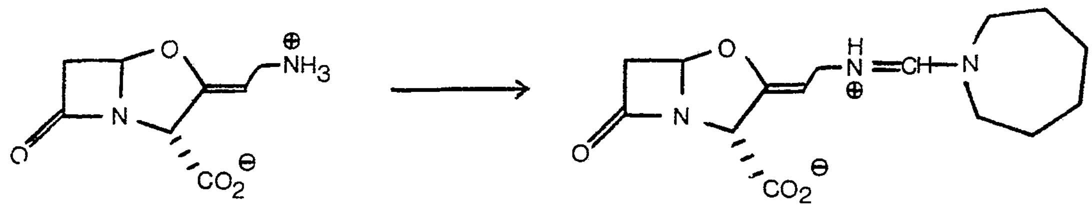

A suspension of 9-ADCA (200 mgs) in MDC (25 mls) was stirred at 0°C during the addition of DBN (140 mgs) followed by ethyl N, N-hexamethylene formimidate tetrafluoroborate (24.3 mgs). Stirring at 0°C was continued for 2 hours before a further addition of DBN (70 mgs) and the formimidate (120 mgs). The solution was allowed to warm to room temperature and stirring continued for 2 hours. The solvent was then evaporated in vacuo.

The residue was chromatographed on silica gel eluting with n-butanol/ethanol/water (1:1:1) to afford the desired product (126mgs, 41%).

$\nu_{max}$ (KBr) 1780, 1700, 1680, 1610 cm $^{-1}$.

δ ($D_2O$), 1.5-1.8 (8H, overlapping m, C$\underline{H}_2$), 3.03 (1H, d, $\underline{J}$ 17Hz, β-lactam C$\underline{H}$H), 3.36 (2H, t, $\underline{J}$ 7Hz, NC$\underline{H}_2$), 3.54 (1H, dd, $\underline{J}$ 17 and 3 Hz, β-lactam CH$\underline{H}$), 3.57 (2H, t, $\underline{J}$ 7Hz, NC$\underline{H}$z), 4.02 (2H, d, $\underline{J}$ 7Hz, C (9) $\underline{H}$), 4.81 (1H, t, $\underline{J}$ 7Hz, vinyl $\underline{H}$), 4.90 (1H, s, C (3) $\underline{H}$), 5.71 (1H, d, $\underline{J}$ 3 Hz, β-lactam C$\underline{H}$), 7.79 (1H, s, N=C$\underline{H}$) ppm.

Example 28

9-N-(1'-amino-2'-methylthioethylidene) amino-9-deoxyclavulanic acid.

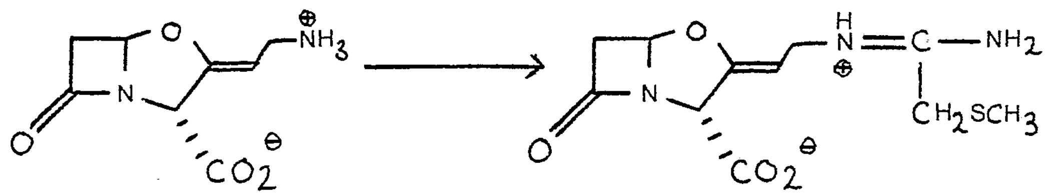

A solution of 9-ADCA (200 mgs) in water (25 mls) was stirred at 0°C and treated with ethyl 2-methylthioacetimidate hydrochloride (680 mgs) added portionwise. During the addition the pH of the solution was maintained at 8.5-9 with 1M LiOH solution dispensed from an automatic burette. The solution was then stirred at pH 9 and 0°C for 1 hour. The reaction mixture was neutralised to pH 6.5 with dil HCl solution before evaporating the solvent in vacuo.

The residue was chromatographed on silica gel eluting with n-butanol/ethanol/water (4:1:1) to afford the required product (127 mgs 44%).

$\nu_{max}$ (KBr) 1780, 1700, 1680, 1600 cm $^{-1}$.

δ ($D_2O$) 2.10 (3H, s, $SC\underline{H}_3$), 3.07 (1H, d, $\underline{J}$ 17Hz, β-lactam C$\underline{H}$H), 3.45 (2H, s, $N{=}CC\underline{H}_2$), ca. 3.5 (1H, β-lactam CH$\underline{H}$), ca. 3.95 (2H, m, C (9) $\underline{H}$), 4.85 (1H, t, $\underline{J}$ 7Hz, vinyl $\underline{H}$), 4.94 (1H, s, C (3) $\underline{H}$), 5.71 (1H, d, $\underline{J}$ 3Hz, β-lactam C$\underline{H}$) ppm.

Example 29

9-N-(1-amino-2-[2'-thienyl] ethylidene) amino-9-deoxyclavulanic acid.

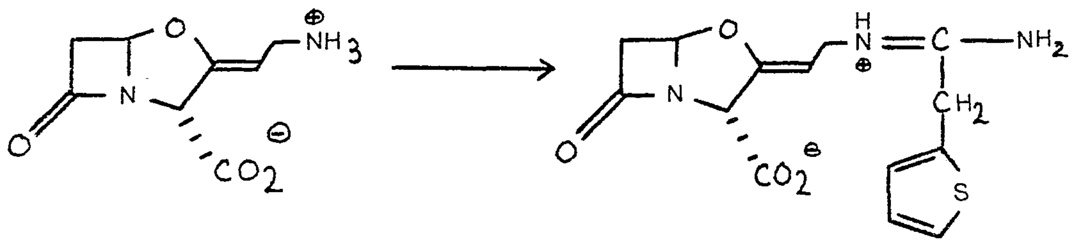

A solution of 9-ADCA (200 mgs) in water (35 mls) was stirred at 0°C and treated with ethyl 2-(2'-thienyl) acetimidate hydrochloride (1.64g) added portionwise. During the addition the pH of the solution was maintained at 8.5-9 with 1M LiOH solution dispensed from an automatic burette. The solution was then stirred at pH 9.5 and 0°C for 1½ hours. The reaction mixture was neutralised to pH 6.5 with dil HCl solution before evaporating the solvent in vacuo.

The residue was chromatographed on silica gel eluting with n-butanol/ethanol/water (4:1:1) to afford the required product (55 mgs, 17%).

$\nu_{max}$ (KBr) 1780, 1700, 1680, 1600 cm $^{-1}$.

δ ($D_2O$) 2.90 (1H, d, J 17Hz, β-lactam CHH), 3.48 (1H, dd, J 17 and 3Hz, β-lactam CHH), 3.89 (2H, d, J 7Hz, C (9) H ), 3.99 (2H, s, N=C$CH_2$), 4.79 (1H, t, J 7Hz, vinyl H), 4.88 (1H, s, C (3) H), 5.62 (1H, d, J 3Hz, β-lactam CH), 7.02 (2H, m, aryl H), 7.39 (1H, m, aryl H) ppm.

Example 30

9-N-(2-pyrrolidylidene) amino-9-deoxyclavulanic acid.

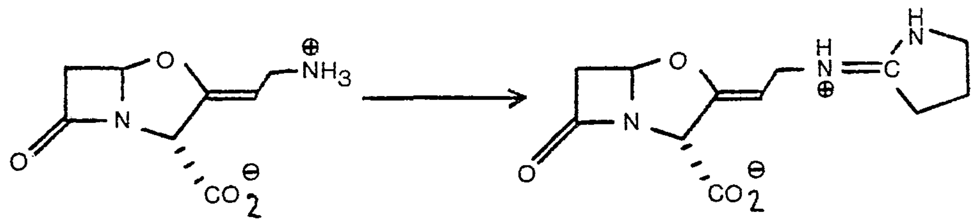

A suspension of 9-ADCA (200 mgs) in MDC (30 mls) was stirred at 0°C during the addition of DBN (140 mgs) followed by 2-ethoxy-1-pyrrolinium tetrafluoroborate (200 mgs). Stirring at 0°C was continued for 1 hour before a further addition of DBN (70 mgs) and the tetrafluoroborate (100 mgs). The solution was allowed to warm to room temperature and stirring continued for 2 hours. The solvent was then evaporated in vacuo.

The residue was chromatographed on silica gel eluting with n-butanol/ethanol/water (2:1:1) to afford the desired product (33 mgs, 12%).

$\nu_{max}$ (KBr) 1785, 1700, 1680, 1610 $cm^{-1}$.

δ ($D_2O$) 1.95 - 2.30 (2H, m, C$\underline{H}_2$), 2.78 (2H, t, $\underline{J}$ 7Hz, N=CC$\underline{H}_2$), 3.03 (1H, d, $\underline{J}$ 17Hz, β-lactam C$\underline{H}$H), 3.53 (1H, dd, $\underline{J}$ 17 and 3Hz, β-lactam CH$\underline{H}$), 3.53 (2H, t, $\underline{J}$ 7Hz, NC$\underline{H}_2$), 3.89 (2H, d, $\underline{J}$ 7Hz, C (9) $\underline{H}$), 4.78 (1H, t, $\underline{J}$ 7Hz, vinyl $\underline{H}$), 4.90 (1H, s, C (3) $\underline{H}$), 5.69 (1H, d, $\underline{J}$ 3Hz, β-lactam C$\underline{H}$), ppm.

Example 31

9-N-(2-piperidylidene) amino-9-deoxyclavulanic acid.

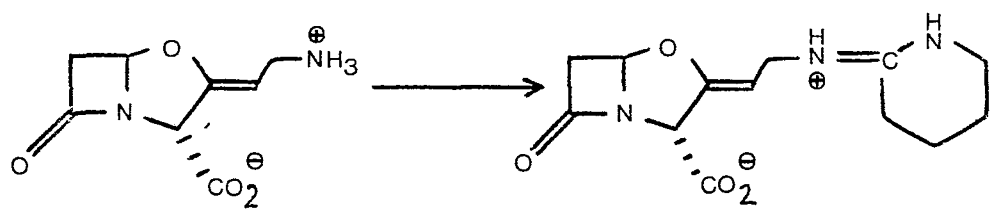

A suspension of 9-ADCA (200mgs) in MDC (30 mls) was stirred at 0°C during the addition of DBN (140 mgs) followed by 2-ethoxy-3,4,5,6-tetrahydropyridinium tetrafluoroborate (215 mgs). Stirring at 0° was continued for 1 hour before a further addition of DBN (70 mgs) and the tetrafluoroborate (108 mgs). The solution was allowed to warm to room temperature and stirring continued for 2 hours. The solvent was then evaporated in vacuo.

The residue was chromatographed on silica gel eluting with n-butanol/ethanol/water (2:1:1) to afford the desired product (48 mgs 17%).

$\nu_{max}$ (KBr) 1785, 1700, 1660, 1610 cm $^{-1}$.

$\delta$ ($D_2O$) 1.7-1.9 (4H, overlapping m, C$\underline{H}_2$), 2.57 (2H, m, N=CC$\underline{H}_2$), 3.10 (1H, d, $\underline{J}$ 17Hz, β-lactam C$\underline{H}$H), 3.40 (2H, m, NC$\underline{H}_2$) 3.60 (1H, dd, $\underline{J}$ 17 and 3Hz, β-lactam CH$\underline{H}$), 3.89 (2H, d, $\underline{J}$ 7Hz, C (9) $\underline{H}$), 4.83 (1H, t, $\underline{J}$ 7Hz, vinyl $\underline{H}$), 4.98 (1H, s, C (3) $\underline{H}$), 5.78 (1H, d, $\underline{J}$ 3Hz, β-lactam C$\underline{H}$) ppm.

Example 32

9-N-[(N-methyl) amino-methylthiomethylene] amino-9-deoxyclavulanic acid.

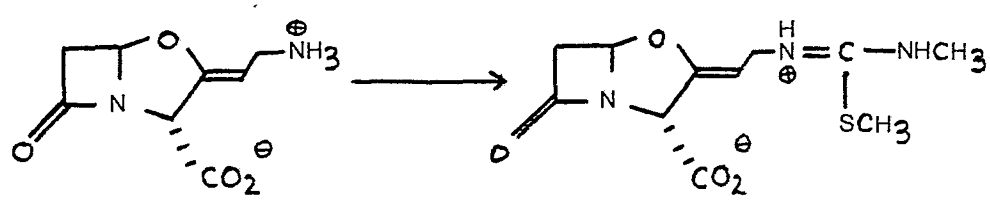

A suspension of 9-ADCA (200mgs) in dry DMF (20 mls) was stirred at 0°C during the addition of DBN (375 mgs), followed by trimethylchlorosilane (109 mgs) and methyl isothiocyanate (77 mgs). The reaction was allowed to warm to room temperature and stir for 4 hours before recooling to 0°C and adding methyl iodide (1 ml). The reaction was allowed to warm to room temperature and stirring continued for a further 3 hours. The reaction mixture was diluted with water (25 mls) and the pH of the solution adjusted to 6 with 1M LiOH solution before evaporating the solvent in vacuo.

The residue was chromatographed on silica gel eluting with n-butanol/ethanol/water (4:1:1) to afford the required product (149 mgs; 52%).

$\nu_{max}$ (KBr) 1780, 1690, 1610 cm $^{-1}$.

δ ($D_2O$) 2.55 (3H, s, SC$\underline{H}_3$), 2.95 (3H, s, NC$\underline{H}_3$), 3.05 (1H, d, $\underline{J}$ 17 Hz, β-lactam C$\underline{H}$H), 3.55 (1H, dd, $\underline{J}$ 17 and 3Hz, β-lactam CH$\underline{H}$), 4.05 (2H, d, $\underline{J}$ 7Hz, C(9)$\underline{H}$), 4.78 (1H, t, $\underline{J}$ 7Hz, vinyl $\underline{H}$), 4.91 (1H, s, C(3)$\underline{H}$), 5.72 (1H, d, $\underline{J}$ 3Hz, β-lactam C$\underline{H}$) ppm.

Demonstration of Effectiveness

In a standard MIC synergy test the following results were obtained.

| Compound of Example | Concentration of inhibitor (µg/ml) | Staph. aureus Russell | Klebsiella Aerogenes E70 | Escherichia coli JT39 |
|---|---|---|---|---|
| 1 | 5.0<br>1.0 | Inhibited<br>2.5 | 3.1<br>12.5 | 16.0<br>62.5 |
| 2 | 5.0<br>1.0 | Inhibited<br>0.6 | (0.8)<br>3.1 | 4.0<br>31.2 |
| 3 | 5.0<br>1.0 | Inhibited<br>0.3 | 0.8<br>3.1 | 2.0<br>31.2 |
| 4 | 5.0<br>1.0 | Inhibited<br>0.15 | 0.8<br>6.2 | 2.0<br>16.0 |
| 5 | 5.0<br>1.0 | Inhibited<br>0.6 | 0.4<br>3.1 | 2.0<br>16.0 |
| 6 | 5.0<br>1.0 | Inhibited<br>Inhibited | 1.6<br>6.2 | (4.0)<br>16.0 |
| For comparison Amoxycillin alone | | 500 | 1000 | 2000 |

Demonstration of Effectiveness

In a standard MIC synergy test the following results were obtained.

| Compound of Example | Concentration of inhibitor (µg/ml) | Staph. aureus Russell | Klebsiella Aerogenes E70 | Escherichia coli JT39 |
|---|---|---|---|---|
| 7 | 5.0<br>1.0 | Inhibited<br>0.6 | 1.6<br>6.25 | 16.0<br>125 |
| 8 | 5.0<br>1.0 | Inhibited<br>1.25 | 50<br>100 | > 500<br>> 500 |
| 9 | 5.0<br>1.0 | 1.25<br>5.0 | 6.2<br>25.0 | 31.2<br>125 |
| 10 | 5.0<br>1.0 | 0.6<br>2.5 | 3.1<br>12.5 | 62.5<br>500 |
| 11 | 5.0<br>1.0 | Inhibited<br>0.6 | 1.6<br>3.1 | 8.0<br>31.2 |
| 12 | 5.0<br>1.0 | Inhibited<br>0.6 | 1.6<br>6.25 | 4.0<br>16.0 |
| For comparison Amoxycillin alone | | 500 | 1000 | 2000 |

Demonstration of Effectiveness

In a standard MIC synergy test the following results were obtained.

| Compound of Example | Concentration of inhibitor (μg/ml) | Staph. aureus Russell | Klebsiella Aerogenes E70 | Escherichia coli JT39 |
|---|---|---|---|---|
| 13 | 5.0<br>1.0 | 0.16<br>0.6 | 1.6<br>6.2 | 8.0<br>31.2 |
| 14 | 5.0<br>1.0 | Inhibited<br>0.3 | 0.8<br>6.2 | 2.0<br>16.0 |
| 16 | 5.0<br>1.0 | Inhibited<br>0.6 | 0.8<br>3.1 | 0.5<br>16.0 |
| 18 | 5.0<br>1.0 | Inhibited<br>0.08 | 0.8<br>3.1 | 4.0<br>8.0 |
| 19 | 5.0<br>1.0 | Inhibited<br>0.08 | 0.8<br>1.5 | Inhibited<br>4.0 |
| 20 | 5.0<br>1.0 | Inhibited<br>0.15 | 0.8<br>3.1 | Inhibited<br>8.0 |
| For comparison Amoxycillin alone | | 500 | 1000 | 2000 |

## Demonstration of Effectiveness

In a standard MIC synergy test the following results were obtained.

| Compound of Example | Concentration of inhibitor (μg/ml) | Staph. aureus Russell | Klebsiella Aerogenes E70 | Escherichia coli JT39 |
|---|---|---|---|---|
| 21 | 5.0<br>1.0 | Inhibited<br>0.6 | 3.1<br>12.5 | 4.0<br>8.0 |
| 22 | 5.0<br>1.0 | Inhibited<br>Inhibited | 0.8<br>6.2 | Inhibited<br>16.0 |
| 23 | 5.0<br>1.0 | Inhibited<br>Inhibited | 3.1<br>12.5 | 4.0<br>31.2 |
| 24 | 5.0<br>1.0 | Inhibited<br>0.6 | 1.6<br>12.5 | 4.0<br>31.2 |
| 25 | 5.0<br>1.0 | Inhibited<br>0.16 | 1.6<br>12.5 | 8.0<br>16.0 |
| 26 | 5.0<br>1.0 | Inhibited<br>Inhibited | 1.6<br>6.2 | 2.0<br>16.0 |
| For comparison Amoxycillin alone | | 500 | 1000 | 2000 |

Demonstration of Effectiveness

In a standard MIC synergy test the following results were obtained.

| Compound of Example | Concentration of inhibitor (μg/ml) | Staph. aureus Russell | Klebsiella Aerogenes E70 | Escherichia coli JT39 |
|---|---|---|---|---|
| 27 | 5.0<br>1.0 | Inhibited<br>Inhibited | 1.5<br>6.25 | 2.0<br>8.0 |
| 28 | 5.0<br>1.0 | 0.04<br>1.25 | 0.8<br>3.1 | 250<br>>500 |
| 29 | 5.0<br>1.0 | 0.08<br>0.3 | 0.4<br>1.5 | 2.0<br>4.0 |
| 30 | 5.0<br>1.0 | Inhibited<br>Inhibited | 3.1<br>12.5 | 8.0<br>62.5 |
| 31 | 5.0<br>1.0 | Inhibited<br>Inhibited | 0.8<br>3.1 | 2.0<br>8.0 |
| | | | | |
| For comparison Amoxycillin alone | | 500 | 1000 | 2000 |

IN A STANDARD ANTIBACTERIAL TEST,
THE FOLLOWING DATA WERE OBTAINED

| Compound of Example | Staph. Aureus Russell |
|---|---|
| 1 | 4.0 |
| 2 | 16.0 |
| 3 | 4.0 |
| 4 | 2.0 |
| 5 | 4.0 |
| 6 | 2.0 |
| 7 | 8.0 |
| 8 | 8.0 |
| 9 | 62.5 |
| 10 | 62.5 |
| 11 | 4.0 |
| 12 | 4.0 |
| 13 | 16.0 |
| 14 | 4.0 |
| 16 | 4.0 |
| 18 | 2.0 |
| 19 | 4.0 |
| 20 | 4.0 |
| 21 | 4.0 |
| 22 | ≤0.5 |
| 23 | 2.0 |
| 24 | 8.0 |
| 25 | 4.0 |
| 26 | ≤0.5 |
| 27 | 1.0 |
| 28 | 2.0 |
| 29 | 2.0 |
| 30 | 1.0 |
| 31 | 1.0 |

CLAIMS

1. A compound of the formula (II):

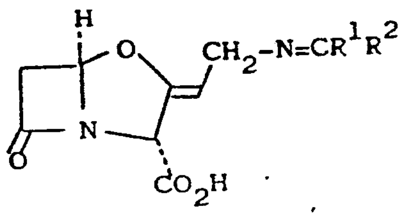

(II)

or a pharmaceutically acceptable salt or ester thereof, or acid addition salt of such ester; wherein

a) $R^1$ is a group $NR^3R^4$ and $R^2$ is a group $NR^5R^6$, $SR^5$, $OR^5$ or $R^5$; or

b) $R^1$ is a group $R^5$ and $R^2$ is either a group $OR^5$ or $SR^5$;

wherein $R^3$ is selected from hydrogen, $C_{1-10}$alkyl, $C_{1-10}$ alkoxy, $C_{2-10}$ alkenyl, $C_{2-10}$ alkynyl, $C_{3-10}$ cycloalkyl, $C_{4-12}$ cycloalkylalkyl, phenyl, phenylalkyl, heteroaryl, heteroaralkyl, heterocyclyl, heterocyclylalkyl or phenylalkoxy; and $R^4$, $R^5$ and $R^6$ are independently selected from the group consisting of hydrogen, $C_{1-10}$ alkyl, $C_{2-10}$ alkenyl, $C_{2-10}$ alkynyl, $C_{3-10}$ cycloalkyl, $C_{4-12}$ cycloalkylalkyl, phenyl, phenylalkyl, heteroaryl, heteroaralkyl, heterocyclyl and heterocyclylalkyl; any of the above $R^3$, $R^4$, $R^5$ and $R^6$ groups optionally being substituted, or $R^3$ and $R^4$ may be joined together to form a heterocyclyl ring, or $R^5$ and $R^6$ may be joined together to form a heterocyclyl ring; or $R^1$ and $R^2$ may be joined together to form a heteroaryl or heterocyclyl ring: with the proviso that $OR^5$ and $SR^5$ are not OH and SH respectively.

2. A compound as claimed in claim 1 wherein $R^3$ is hydrogen, $C_{1-10}$ alkyl, $C_{2-10}$ alkenyl, $C_{2-10}$ alkynyl, $C_{3-10}$ cycloalkyl, $C_{4-12}$ cycloalkylalkyl, phenyl, phenylalkyl, heteroaryl, heteroaralkyl, heterocyclyl or heterocyclylalkyl.

3. A compound as claimed in either claim 1 or claim 2 of the formula (IV):

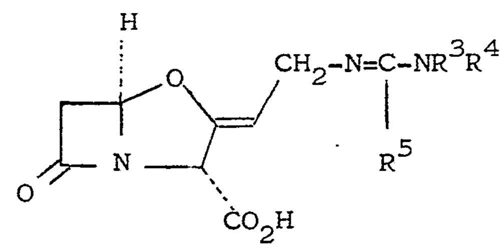

(IV)

or a pharmaceutically acceptable ester thereof or pharmaceutically acceptable acid addition salt of such an ester.

4. A compound as claimed in claim 3 wherein $R^3$, $R^4$ and $R^5$ are independently hydrogen, $C_{1-6}$ alkyl, phenyl or benzyl.

5. A compound as claimed in either claim 3 or claim 4 wherein $R^3$ and $R^4$ are each hydrogen.

6. A compound selected from: 9-N-(dimethylaminomethylene)amino-9-deoxyclavulanic acid, 9-N-(1'-aminoethylidene)amino-9-deoxyclavulanic acid, 9-N-[1'-(N-isobutyl)aminoethylidene]amino-9-deoxyclavulanic acid, 9-N-[1'-(N-benzyl)aminoethylidene]amino-9-deoxyclavulanic acid, 9-N-(aminomethylene)amino-9-deoxyclavulanic acid, 9-N-(N-benzylaminomethylene)amino-9-deoxyclavulanic acid, 9-N-(1-aminobenzylidene)amino-9-deoxyclavulanic acid, 9-N-[1-(N-5'-benzyloxycarbonylpentyl)aminoethylidene]-amino-9-deoxyclavulanic acid, potassium 9-N-[1'-(N-carboxylatomethyl)aminoethylidene]amino-9-deoxyclavulanic acid, 9-N-(N-methylaminomethylene)amino-9-deoxyclavulanic acid, 9-N-(1-aminobutylidene)amino-9-deoxyclavulanic acid, 9-N-(1'-amino-3'-methoxypropylidene)amino-9-deoxyclavulanic acid, 9-N-(1'-aminopropylidene)amino-9-deoxyclavulanic acid, 9-N-(1'amino-2'-methylpropylidene)amino-9-deoxyclavulanic acid, 9-N-(1'-amino-3'-methylbutylidene)amino-9-deoxyclavulanic acid, 9-N-(1-aminopentylidene)amino-9-deoxyclavulanic acid, 9-N-(aminocyclopropylmethylene)amino-9-deoxyclavulanic acid, 9-N-(aminocyclobutylmethylene)amino-9-deoxyclavulanic acid, 9-N-(diethylaminomethylene)amino-9-deoxyclavulanic acid, 9-N-[1-(N,N-dimethyl)aminoethylidene]amino-9-deoxyclavulanic acid, 9-N-[1'-(N-methyl)aminobenzylidene]amino-9-deoxyclavulanic acid, 9-N-[1-(N-methyl)aminoethylidene]amino-9-deoxyclavulanic acid, 9-N-[1-(N-ethyl)aminoethylidene]-amino-9-deoxyclavulanic acid, 9-N-(N,N-pentamethylene aminomethylene)amino-9-deoxyclavulanic acid, 9-N-(N,N-hexamethyleneaminomethylene)amino-9-deoxyclavulanic acid, 9-N-(1'-amino-2'-methylthioethylidene)amino-9-deoxyclavulanic acid, 9-N-(1-amino-2-[2'-thienyl]ethylidene) amino-9-deoxyclavulanic acid, 9-N-(2-pyrrolidylidene)amino-9-deoxyclavulanic acid, 9-N-(2-piperidylene)amino-9-deoxyclavulanic acid, and 9-N-[(N-methyl)aminomethylthiomethylene]amino-9-deoxyclavulanic acid.

7. A compound as claimed in either claim 1 or 2 of the formula (V):

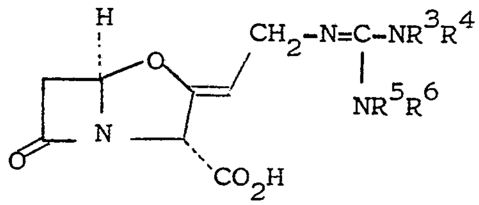

(V)

or a pharmaceutically acceptable ester thereof or pharmaceutically acceptable acid addition salt of such an ester.

8. A compound as claimed in either claim 1 or 2 of the formula (VI):

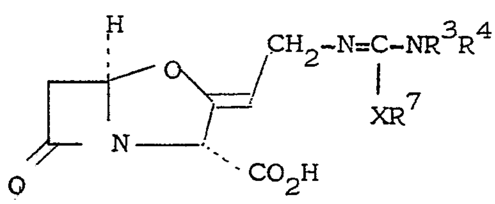

(VI)

or a pharmaceutically acceptable ester thereof or pharmaceutically acceptable acid addition salt of such an ester, wherein X is an oxygen or sulphur atom and $R^7$ is of the value $R^5$ as defined in claim 1 except that it is not a hydrogen atom.

9. A compound as claimed in either claim 1 or claim 2 of the formula (VII):

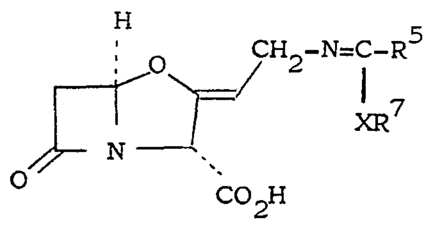

(VII)

or a pharmaceutically acceptable salt or ester thereof or pharmaceutically acceptable acid addition salt of such an ester, wherein X is an oxygen or sulphur atom and $R^7$ is of the value $R^5$ as defined in claim 1 except that it is not a hydrogen atom.

10. A pharmaceutical composition comprising a compound as claimed in any of claims 1 to 9 and a pharmaceutically acceptable carrier.

11. A pharmaceutical composition as claimed in claim 10 which also comprises a penicillin or cephalosporin.

12. A process for the preparation of a compound of the formula (IV) or (V):

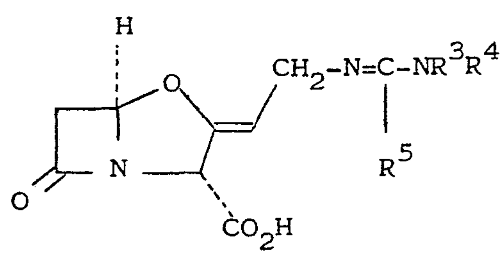

(IV)

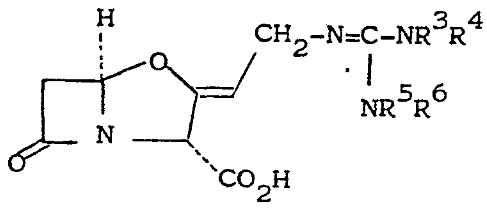

(V)

or a pharmaceutically acceptable ester thereof or pharmaceutically acceptable acid addition salt of such an ester, wherein $R^3$ is selected from hydrogen, $C_{1-10}$ alkyl, $C_{1-10}$ alkoxy, $C_{2-10}$ alkenyl, $C_{2-10}$ alkynyl, $C_{3-10}$ cycloalkyl, $C_{4-12}$ cycloalkylalkyl, phenyl, phenylalkyl, heteroaryl, heteroaralkyl, heterocyclyl, heterocyclylalkyl or phenylalkoxy; and $R^4$, $R^5$ and $R^6$ are independently selected from the group consisting of hydrogen, $C_{1-10}$ alkyl, $C_{2-40}$ alkenyl, $C_{2-10}$ alkynyl, $C_{3-10}$ cycloalkyl, $C_{4-12}$ cycloalkylalkyl, phenyl, phenylalkyl, heteroaryl, heteroaralkyl, heterocyclyl or heterocyclylalkyl, any of the above $R^3$, $R^4$, $R^5$ and $R^6$ groups being optionally substituted; or $R^3$ and $R^4$ may be joined together to form a heterocyclyl ring, or $R^5$ and $R^6$ may be joined together to form a heterocyclyl ring: which process comprises either:

i) the reaction of a compound of the formula (X) or a derivative thereof that allows alkylation to occur:

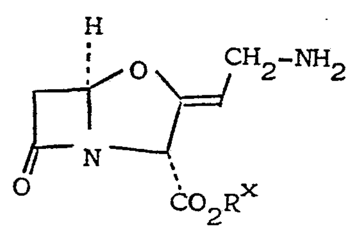

(X)

wherein $R^x$ is a hydrogen atom or a blocking group; with either the compound of the formula (XI) or (XII):

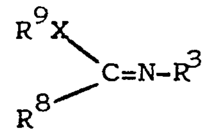

(XI)

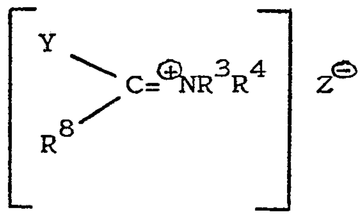

(XII)

wherein $R^8$ is a group $R^5$ or $NR^5R^6$; $R^9$ is $C_{1-6}$ alkyl, X is an oxygen or sulphur atom, Y is halo or $C_{1-6}$ alkoxy, and Z is a salting-ion:

or ii) the reaction of a compound of the formula (XIII):

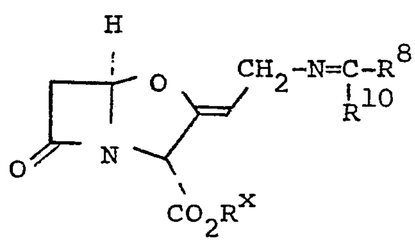

(XIII)

wherein $R^x$ and $R^8$ are as hereinbefore defined and $R^{10}$ is chloro or a group $XR^9$ as hereinbefore defined; with a compound of the formula (XIV):

$NHR^3R^4$ (XIV)

and thereafter if necessary removing the blocking group, forming an ester or forming an acid addition salt of an ester.

13. A process for the preparation of a compound of the formula (VI):

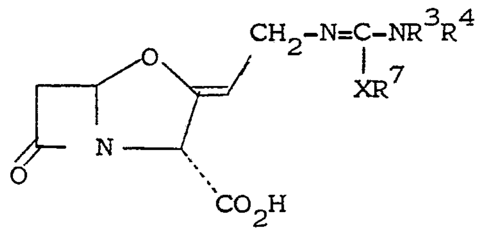

(VI)

or a pharmaceutically acceptable ester thereof or pharmaceutically acceptable acid addition salt of such an ester, wherein X is an oxygen or sulphur atom, $R^7$ is of value $R^5$ except that it is not a hydrogen atom, and $R^3$, $R^4$ and $R^5$ are as defined in claim 12: which process comprises the reaction of a compound of the formula (XV):

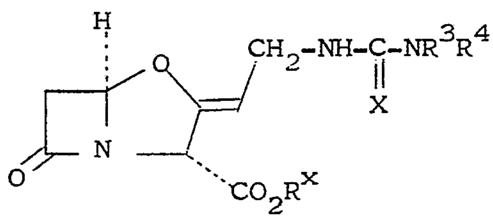

(XV)

wherein $R^x$ is a hydrogen atom or blocking group, and a reactive alkylating agent; and thereafter if necessary, removing the blocking group, forming an ester or forming an acid addition salt of an ester.

14. A process for the preparation of a compound of the formula (VII):

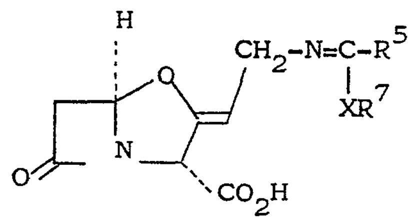

(VII)

or a pharmaceutically acceptable salt or ester thereof or pharmaceutically acceptable acid addition salt of such an ester, wherein X is an oxygen or sulphur atom, $R^7$ is of the value $R^5$ except that it is not a hydrogen atom, and $R^5$ is as defined in claim 12; which process comprises the reaction of a compound of the formula (XVI):

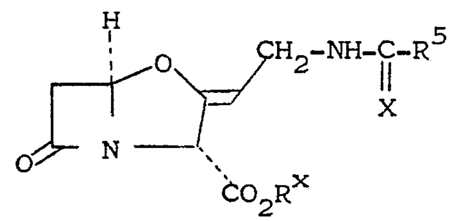

(XVI)

wherein $R^x$ is a hydrogen atom or blocking group, and a reactive alkylating agent; and thereafter if necessary removing the blocking group, forming an ester or salt, or forming an acid addition salt of an ester.

European Patent Office

# EUROPEAN SEARCH REPORT

Application number
EP 81 30 3534

| DOCUMENTS CONSIDERED TO BE RELEVANT | | | CLASSIFICATION OF THE APPLICATION (Int. Cl.) |
|---|---|---|---|
| Category | Citation of document with indication, where appropriate, of relevant passages | Relevant to claim | |
| | EP - A - 0 005 014 (BEECHAM) * pages 57 and 59 * | 1,12 | C 07 D 498/04<br>A 61 K 31/42//<br>(C 07 D 498/04<br>263/00<br>205/00<br>A 61 K 31/42<br>31/43<br>31/42<br>31/545) |
| | EP - A - 0 010 369 (BEECHAM) * page 21 * | 1,12 | |
| | EP - A - 0 001 333 (BEECHAM) * claims 1,22-29 * | 1,10-12 | |
| | | | TECHNICAL FIELDS SEARCHED (Int. Cl.) |
| | | | C 07 D 498/04<br>A 61 K 31/42 |
| | | | CATEGORY OF CITED DOCUMENTS<br>X: particularly relevant<br>A: technological background<br>O: non-written disclosure<br>P: intermediate document<br>T: theory or principle underlying the invention<br>E: conflicting application<br>D: document cited in the application<br>L: citation for other reasons |
| X | The present search report has been drawn up for all claims | | &: member of the same patent family, corresponding document |

| Place of search | Date of completion of the search | Examiner |
|---|---|---|
| The Hague | 02-11-1981 | CHOULY |